# EUROPEAN PATENT APPLICATION

(11) **EP 4 202 049 A1**
(43) Date of publication of application: **28.06.2023**
(21) Application number: 22204880.3
(22) Date of filing: 20.09.2018
(51) Int. Cl.: C12P 1/00, C12N 5/07

(54) **PRODUCTION OF EXTRACELLULAR VESICLES IN SUSPENSION USING CHEMICALLY-DEFINED CELL CULTURE MEDIA**

(30) Priority: 21.09.2017 US 201762561206 P; 07.05.2018 US 201862668217 P
(62) Divisional of application: 18858922.0
(71) Applicant: Codiak BioSciences, Inc., Cambridge, MA 02140 (US)
(72) Inventor: VILLIGER, Agata A., Cambridge, 02140 (US); GRUBE, Andrew F., Cambridge, 02140 (US); CHAN, Tik Yan, Cambridge, 02140 (US); ESTES, Scott D., Cambridge, 02140 (US); GOLDEN, Kathryn E., Cambridge, 02140 (US); KONSTANTINOV, Konstantin, Cambridge, 02140 (US); WILLIAMS, Douglas E., Cambridge, 02140 (US)
(74) Representative: Carpmaels & Ransford LLP

(57) **Abstract**

Described herein are methods for the production of extracellular vesicles comprising culturing a population of producer cells in single-cell suspension, wherein the cells are cultured in chemically-defined culture medium, wherein the culture medium lacks animal-derived serum and animal-derived components; and obtaining from the cell culture an extracellular vesicle preparation comprising extracellular vesicles. In certain embodiments, the methods comprise perfusion culturing methods, including single-cell perfusion culturing methods and batch-refeed culturing methods. The methods described herein are a significant improvement over the state of the art and fulfills an unmet need in the field of extracellular vesicle manufacturing and quality control.

## Description

### CROSS-REFERENCE TO RELATED APPLICATIONS

This application claims the benefit of U.S. Provisional Application Nos. 62/561,206, filed September 21, 2017; and 62/668,217, filed May 7, 2018, each of which is incorporated in its entirety by reference.

### BACKGROUND

### Description of the Related Art

In recent years, extracellular vesicles, in particular exosomes, have been gaining interest as a new modality capable of an efficient delivery of various payloads to cells of all types within a living organism. While the exact nature and mechanism of this action is still under investigation, it has been recognized that high titer production of exosomes is necessary to exert the desired biological and clinical effect (Cheng and Schorey, 2016, Biotech Bioeng 113(6): 1315-1324; Kalluri, 2016, J Clin Invest 126(4): 1208-1215). As efforts accelerate to translate exosome biology into new medicines, technology gaps have emerged between the current state of the art for producing exosomes and the capabilities necessary to support large scale clinical and commercial manufacturing. To that end, considerable attempts have been focused on sustaining growth and productivity of the producer cell line *in vitro,* however, maximizing exosome yield in a suspension cell culture process devoid of any animal-derived components at every process stage remains a challenge (Whitford et al., 2015, GEN 35(16)). In addition, although clinical-grade exosomes have been previously generated, they were most commonly produced from cancer patient's blood, or adherent cultures of cells grown in media supplemented with animal serum, at least in the growth phase (Chaput and Thery, 2011, Semin Immunopathol (2011) 33:419-440; Dai et al., 2008, Mol Ther 16(4):782-790; Escudier et al., 2005, J Transl Med 3(1): 10; Morse et al., 2005, J Transl Med 3(1):9).

Therefore, novel methods for efficient, low-cost and reliable high titer production of extracellular vesicles in the absence of animal-derived components are needed.

### SUMMARY

In certain aspects, disclosed herein are methods for the production of extracellular vesicles comprising culturing a population of producer cells in single-cell suspension, wherein the cells are cultured in chemically defined culture medium, wherein the culture medium lacks animal-derived serum and animal-derived components; and obtaining from the cell culture an extracellular vesicle preparation comprising extracellular vesicles. In certain embodiments, the viability of the cells after 8 days in culture is greater than 98%. In certain embodiments, the cell viability is greater than 90% after 21 days in culture. In certain embodiments, the cell viability is greater than 95% after 21 days in culture. In certain embodiments the volumetric productivity of extracellular vesicles is greater than 1 × 10¹⁰ extracellular vesicle particles/ml culture /day. In certain embodiments, the volumetric productivity of extracellular vesicles is greater than 1 × 10¹¹ extracellular vesicle particles/ml culture /day. In certain embodiments, the yield of extracellular vesicles is greater than 3 × 10¹⁵ extracellular vesicle particles/2.4L reactor volume/12-day culture duration. In certain embodiments, the culturing is performed using a fed-batch culturing method. In certain embodiments, the culturing is performed using a batch refeed culturing method. In certain embodiments, the culturing is performed using a perfusion culturing method. In certain embodiment, the perfusion culturing method is tangential flow filtration perfusion. In certain embodiments, the perfusion culturing method is alternating tangential flow filtration perfusion. In certain embodiments, the method results in increased cell viability compared to cells cultured using a fed-batch culturing method cultured for the same number of days. In certain embodiments, the increased cell viability is two-fold or greater cell viability after 10 days of culture. In certain embodiments, the extracellular vesicles are fractionated by column chromatography to determine the amount of contaminants in the isolated extracellular vesicles. In certain embodiments, the extracellular vesicle preparation comprises reduced proteinaceous contaminants, nucleic acid contaminants, small molecules, metabolites, membranous contaminants, or combinations thereof, compared to extracellular vesicle preparations harvested using a fed-batch culturing method cultured for the same number of days. In certain embodiments, the reduced proteinaceous contaminants, nucleic acid contaminants, small molecules, metabolites, membranous contaminants, or combinations thereof are less than 60% for first 10 days of culture. In certain embodiments, the proteinaceous contaminants are measured by ratio of 280 nm / 350 nm signal. In certain embodiments, the nucleic acid contaminants are measured by 254 nm signal. In certain embodiments, the extracellular vesicle preparation comprises increased abundance of extracellular vesicles, compared to extracellular vesicle preparations harvested using a fed-batch culturing method cultured for the same number of days. In certain embodiments, the increased abundance of extracellular vesicles is greater than 4-fold after 9 days of culture. In certain embodiments, the cells are cultured in a culturing vessel. In certain embodiments, the culturing vessel is a shake flask, a conical tube, a shallow-well multititer plate, a deep-well multititer plate, or a bioreactor. In certain embodiments, the culturing vessel is a bioreactor. In certain embodiments, the bioreactor is a microbioreactor, a glass bioreactor, a stainless steel bioreactor, or a single use bioreactor. In certain embodiments, the bioreactor is a perfusion bioreactor. In certain embodiments, the bioreactor is connected to a cell retention device. In certain embodiments, the bioreactor is connected to a cell retention device with filtering technology. In certain embodiments, the cells are subjected to mixing during culture. In certain embodiments, the mixing is shaking, agitation, or stirring. In certain embodiments, the shaking is performed at a speed of 10 rpm or greater. In certain embodiments, the shaking is performed at a speed of 125 rpm. In certain embodiments, the shaking is performed at a speed of 160 rpm. In certain embodiments, the shaking is performed at a speed of 220 rpm. In certain embodiments, the cells are shaken at an angle of 90 degrees. In certain embodiments, the cells are shaken at an angle less than 90 degrees. In certain embodiments, the shaking speed is 160 rpm and cells are shaken at an angle less than 90 degrees. In certain embodiments, the extracellular vesicle preparation is fractionated by a chromatography step, wherein the fractions are detected by intrinsic fluorescence of the extracellular vesicles. In certain embodiments, the intrinsic fluorescence is detected using an excitation wavelength ranging from 450 nm to 650 nm. In certain embodiments, the intrinsic fluorescence is detected using an excitation wavelength of 556 nm. In certain embodiments, the intrinsic fluorescence is detected using an emission wavelength ranging from 500 nm to 650 nm. In certain embodiments, the intrinsic fluorescence is detected using an emission wavelength of 573 nm. In certain embodiments, the chromatography is size-exclusion chromatography. In certain embodiments, the chromatography is ion-exchange chromatography. In certain embodiments, the ion-exchange chromatography is strong anion-exchange chromatography. In certain embodiments, the peak fraction from the chromatography step is isolated. In certain embodiments, the cells are mammalian cells. In certain embodiments, the cells are Chinese hamster ovary (CHO) cells. In certain embodiments, the cells are human cells. In certain embodiments, the human cells are human kidney cells. In certain embodiments, the cells are HEK293 cells. In certain embodiments, the cells are HEK293 SF cells. In certain embodiments, the cells are insect cells. In certain embodiments, the cells are yeast cells. In certain embodiments, the cells are bacteria cells. In certain embodiments, the cells overexpress an exosome specific protein, thereby generating engineered extracellular vesicles overexpressing the exosome specific protein. In certain embodiments, the exosome-specific protein is PTGFRN, BSG, IGSF2, IGSF3, IGSF8, ITGB1, ITGA4, SLC3A2, or ATP transporter, or a fragment or variant thereof. In certain embodiments, the exosome-specific protein is PTGFRN or a fragment or variant thereof. In certain embodiments, the exosome-specific protein is MARCKS, MARCKSL1, or BASP1, or a fragment or variant thereof. In certain embodiments, the extracellular vesicles comprise at least one therapeutic agent. In certain embodiments, the extracellular vesicles are exosomes.

### BRIEF DESCRIPTION OF THE SEVERAL VIEWS OF THE DRAWINGS

These and other features, aspects, and advantages of the present invention will become better understood with regard to the following description, and accompanying drawings, where:

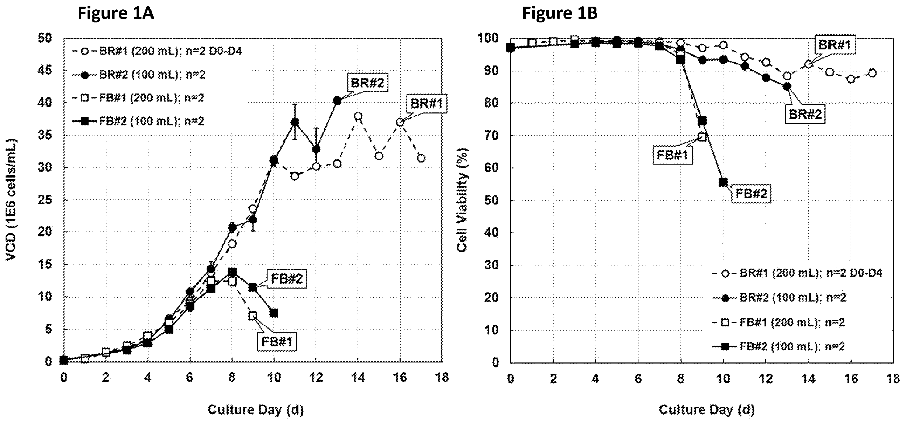

**Figure 1A** is a graph showing viable cell density for batch-refeed (BR) and fed-batch (FB) cultures grown in shake flasks over time. Error bars represent standard deviation.
**Figure 1B** is a graph showing cell viability for batch-refeed (BR) and fed-batch (FB) cultures in shake flasks over time. Error bars represent standard deviation.

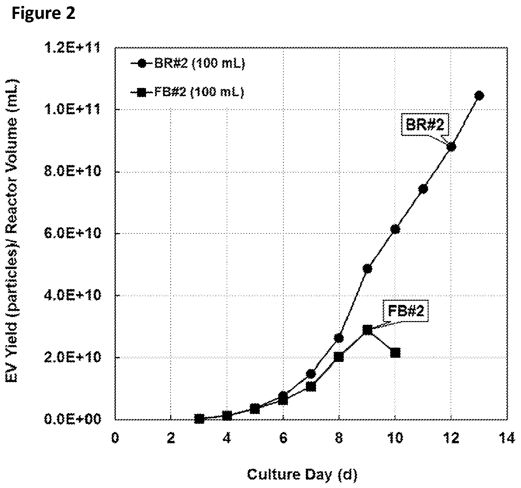

**Figure 2** is a graph showing extracellular vesicle (EV) yield from 100 mL batch-refeed (BR) and fed-batch (FB) cultures grown in shake flasks, represented by particle accumulation over time, and where particle concentration is measured by nanoparticle tracking analysis (NTA) and approximated based on standard curve of NTA plotted as a function of peak area.

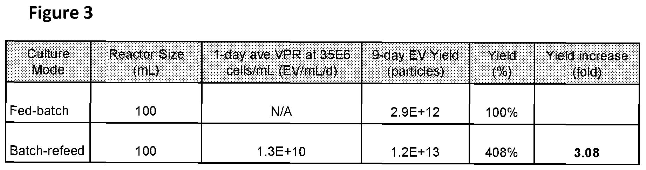

**Figure 3** is a table showing culture productivity in different operating modes for 100 mL batch-refeed (BR#2) and fed-batch (FB#2) cultures. Nine-day exosome yield in batch refeed is calculated based on the average volumetric productivity (VPR) measured at ~35E6 cells/mL.

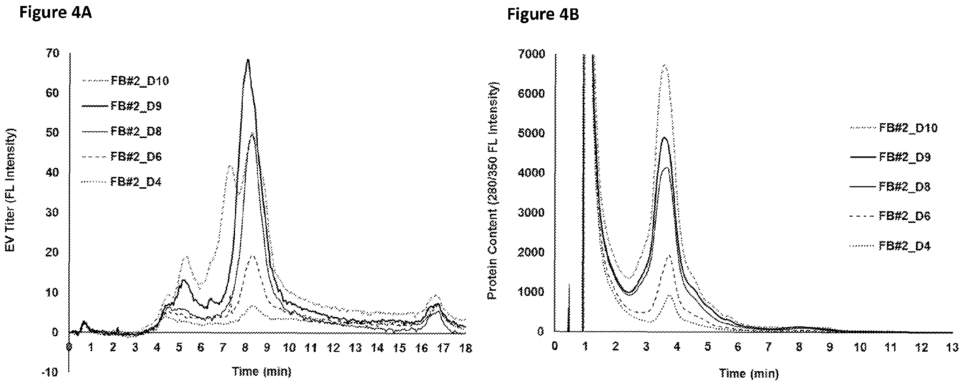

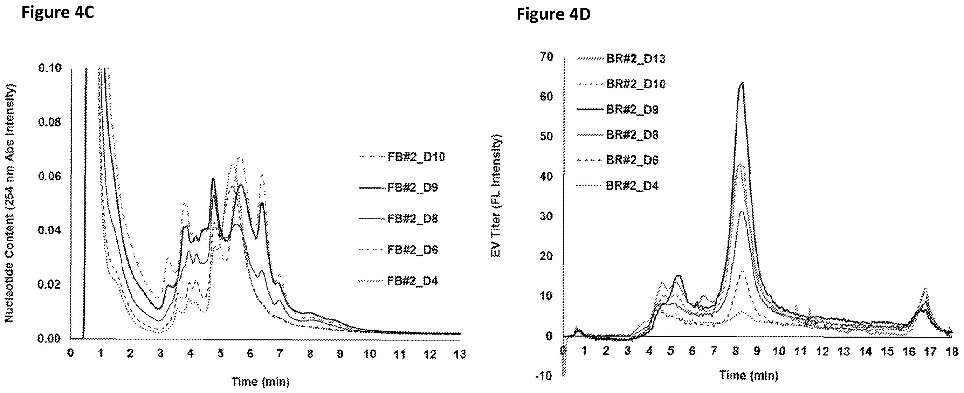

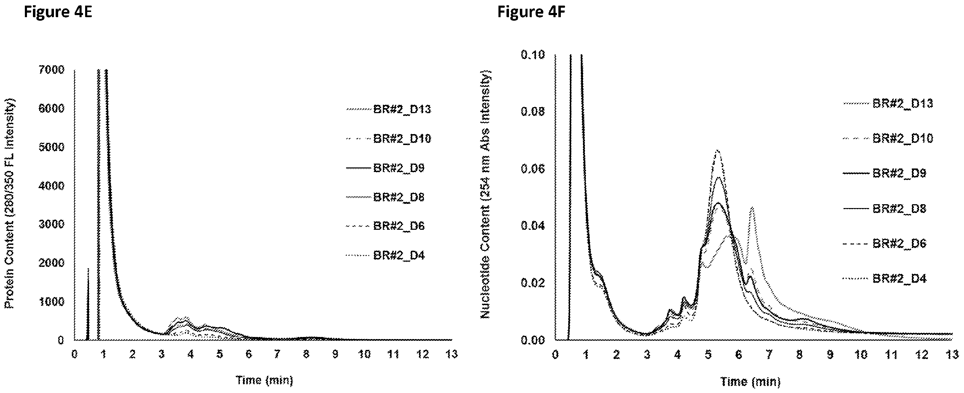

**Figure 4A** is a graph showing AEX HPLC chromatograms describing EV titer in the culture harvest of fed-batch (FB#2) shake flask cultures over time. **Figure 4B** is a graph showing AEX HPLC chromatograms describing content of proteinaceous contaminants, including small molecules, metabolites, membranous contaminants, and combinations thereof in the culture harvest of fed-batch (FB#2) shake flask cultures over time. **Figure 4C** is a graph showing AEX HPLC chromatograms describing content of nucleic acid contaminants and other molecules besides proteins, such as small molecules, metabolites, membranous contaminants and combination thereof in the culture harvest of fed-batch (FB#2) shake flask cultures over time. **Figure 4D** is a graph showing AEX HPLC chromatograms describing EV titer in the culture harvest of batch-refeed (BR#2) shake flask cultures over time. **Figure 4E** is a graph showing AEX HPLC chromatograms describing content of proteinaceous contaminants, including small molecules, metabolites, membranous contaminants, and combinations thereof in the culture harvest of batch-refeed (BR#2) shake flask cultures over time. **Figure 4F** is a graph showing AEX HPLC chromatograms describing content of nucleic acid contaminants and other molecules besides proteins, such as small molecules, metabolites, membranous contaminants and combination thereof in the culture harvest of batch-refeed (BR#2) shake flask cultures over time.

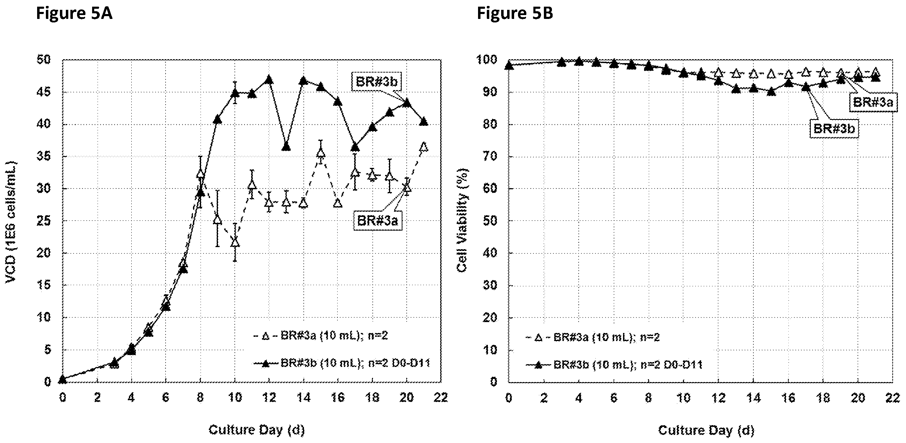

**Figure 5A** is a graph showing viable cell density over time for batch-refeed (BR) cultures maintained at ~30E6 cells/mL (BR#3a) and 40E6 cells/mL (BR#3b), respectively, upon which daily cell bleeds were implemented to maintain the cultures at around the corresponding target density. Error bars represent standard deviation. **Figure 5B** is a graph showing cell viability over time for batch-refeed (BR) cultures maintained at ~30E6 cells/mL (BR#3a) and 40E6 cells/mL (BR#3b), respectively, upon which daily cell bleeds were implemented to maintain the cultures at around the corresponding target density. Error bars represent standard deviation.

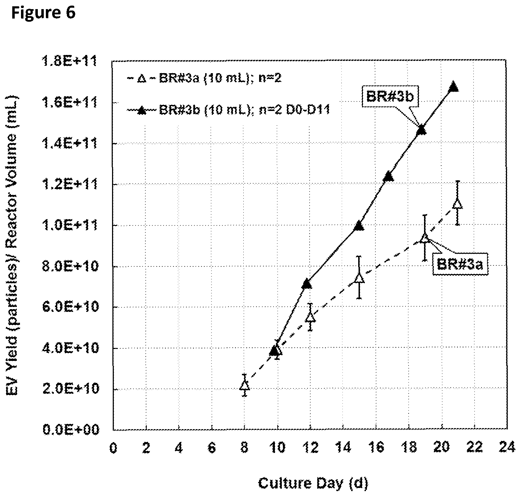

**Figure 6** is a graph showing EV yield from 10 mL batch-refeed (BR) cultures grown in spin tubes maintained for 12 to 14 days at 30E6 cells/mL (BR#3a) and 40E6 cells/mL (BR#3b), respectively, and represented by particle accumulation over time during the steady-state, where particle concentration is measured by nanoparticle tracking analysis (NTA) and approximated based on standard curve of NTA plotted as a function of peak area. The exosome-containing culture harvest was collected daily from the quasi steady-state cultures maintained at the respective cell densities for 12 (BR#3b) to 14 (BR#3a) days. BR#3a: The error bars represent standard deviation.

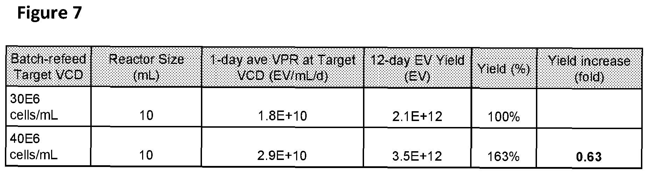

**Figure 7** is a table showing culture productivity in 10 mL simulated perfusion, batch-refeed (BR) maintained at 30E6 cells/mL (BR#3a) and 40E6 cells/mL (BR#3b) with cell bleeds. Twelve-day exosome yield is calculated based on the average volumetric productivity (VPR) measured at the respective target density.

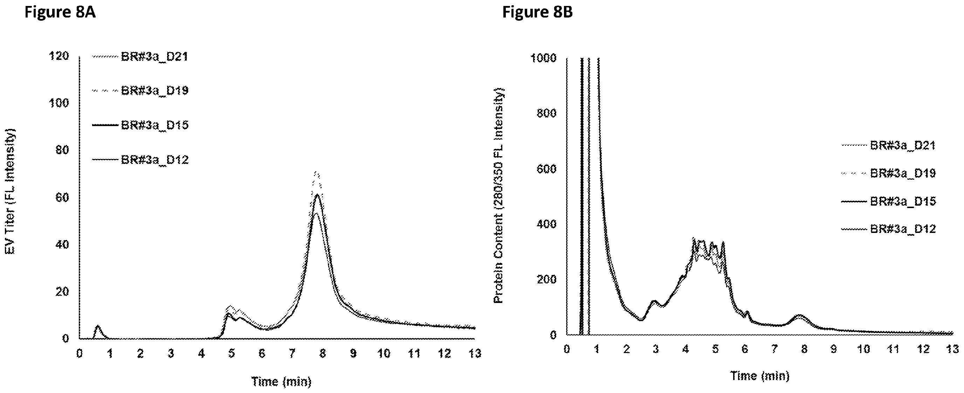

**Figure 8A** is a graph showing AEX HPLC chromatograms describing EV titer for batch-refeed culture maintained at 30E6 cells/mL (BR#3a) in spin tubes with cell bleeds.

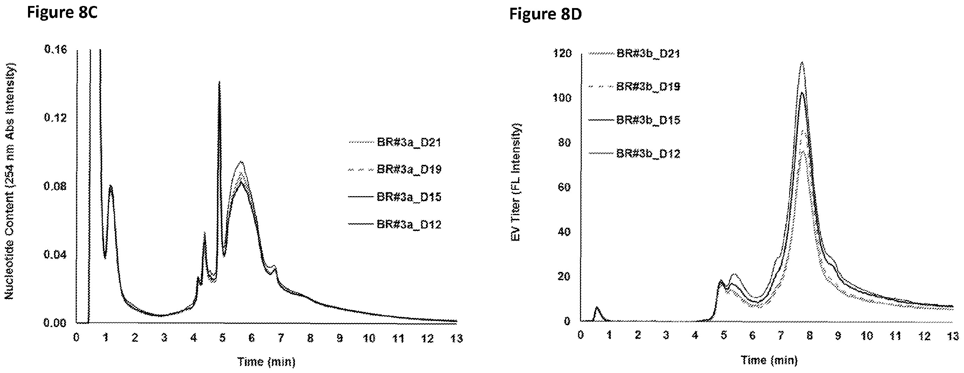

**Figure 8B** is a graph showing AEX HPLC chromatograms describing content of proteinaceous contaminants, including small molecules, metabolites, membranous contaminants, and combinations thereof for batch-refeed culture maintained at 30E6 cells/mL (BR#3a) in spin tubes with cell bleeds. **Figure 8C** is a graph showing AEX HPLC chromatograms describing content of nucleic acid contaminants and other molecules besides proteins, such as small molecules, metabolites, membranous contaminants and combination thereof for batch-refeed culture maintained at 30E6 cells/mL (BR#3a) in spin tubes with cell bleeds. **Figure 8D** is a graph showing AEX HPLC chromatograms describing EV titer for batch-refeed culture maintained at 40E6 cells/mL (BR#3b) in spin tubes with cell bleeds.

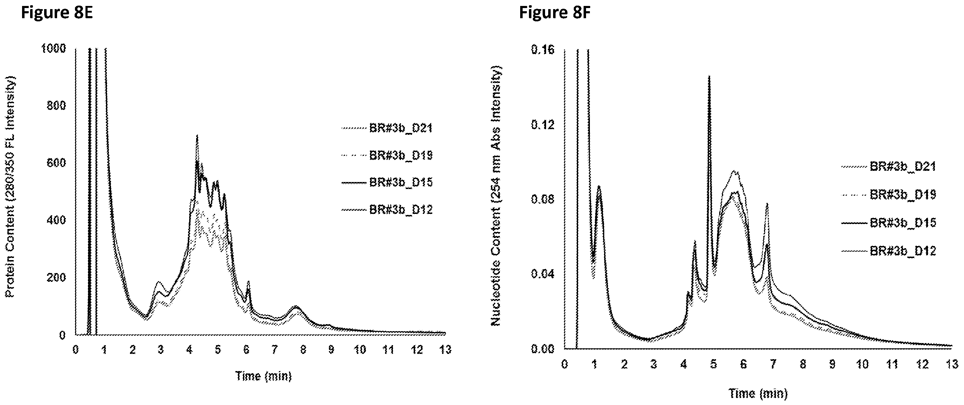

**Figure 8E** is a graph showing AEX HPLC chromatograms describing content of proteinaceous contaminants, including small molecules, metabolites, membranous contaminants, and combinations thereof for batch-refeed culture maintained at 40E6 cells/mL (BR#3b) in spin tubes with cell bleeds. **Figure 8F** is a graph showing AEX HPLC chromatograms describing content of nucleic acid contaminants and other molecules besides proteins, such as small molecules, metabolites, membranous contaminants and combination thereof for batch-refeed culture maintained at 40E6 cells/mL (BR#3b) in spin tubes with cell bleeds.

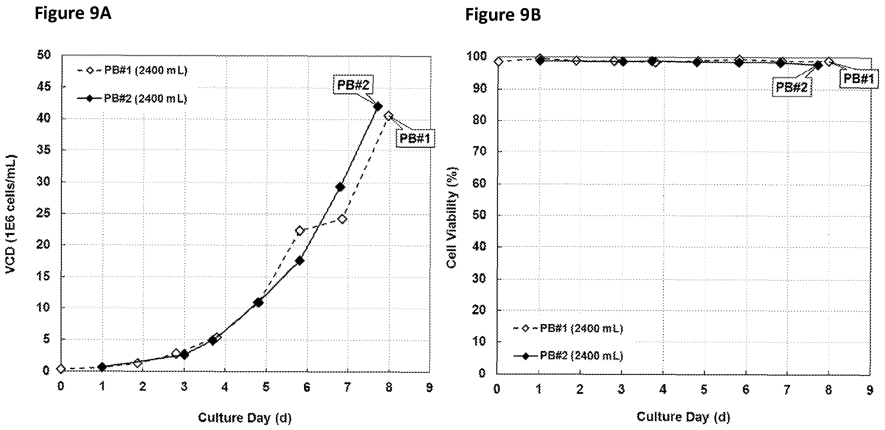

**Figure 9A** is a graph showing viable cell density over time for perfusion bioreactor (PB) cultures PB#1 and PB#2 run in alternating tangential flow filtration (ATF) mode.
**Figure 9B** is a graph showing cell viability over time for perfusion bioreactor (PB) cultures PB#1 and PB#2 grown in bench-top bioreactors. For both PB#1 and PB#2, perfusion was started on culture day 3 at 1 RV/d. During perfusion, culture harvest containing EVs (exosomes) was collected through a filtering device, while the cells were retained in the bioreactor.

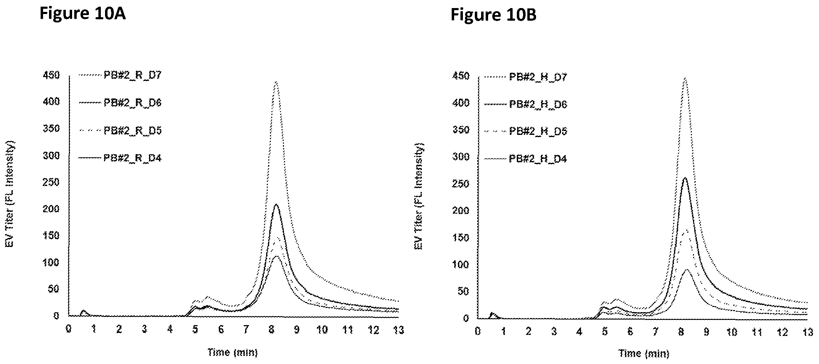

**Figure 10A** is a graph showing AEX HPLC chromatograms describing EV titer for perfusion bioreactor (PB#2) culture samples pulled over time directly from the reactor (R).
**Figure 10B** is a graph showing AEX HPLC chromatograms describing EV titer for perfusion bioreactor (PB#2) culture samples pulled over time directly from the clarified harvest (H) post-filter. **Figure 10A** and **Figure 10B** show almost complete passage of exosomes through the filter and into the harvest, with no apparent exosome retention inside the bioreactor.

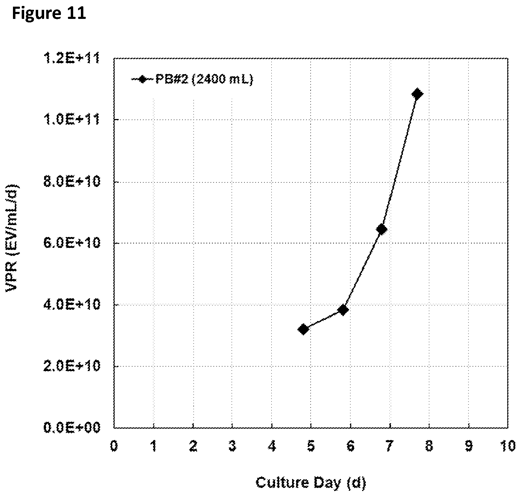

**Figure 11** is a graph showing EV volumetric productivity (VPR) in 2.4 L perfusion bioreactor (PB#2) culture grown in a bench-top bioreactor, and represented by particle production rate over time, where particle concentration is measured by nanoparticle tracking analysis (NTA) and approximated based on standard curve of NTA plotted as a function of peak area. The exosome-containing culture harvest was collected daily starting from ~D4 from the exponentially-growing culture, and until the target cell density of 40E6 cells/mL was reached. Based on day 8 productivity at 40E6 cells/mL, EV yield from a 12-day perfusion bioreactor culture at 2.4 L working volume is estimated. Based on the VPR of ∼1.1E10 EV/mL/d measured at ~40E6 cells/mL, 12-day exosome yield from a 2.4L reactor amounts to ∼3E15 particles.

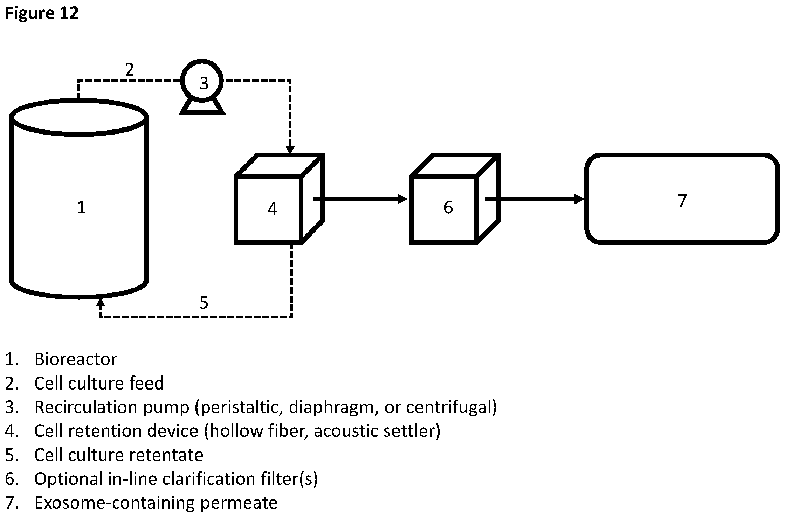

**Figure 12** is a drawing showing the schematic for a tangential flow filtration perfusion cell culture system.

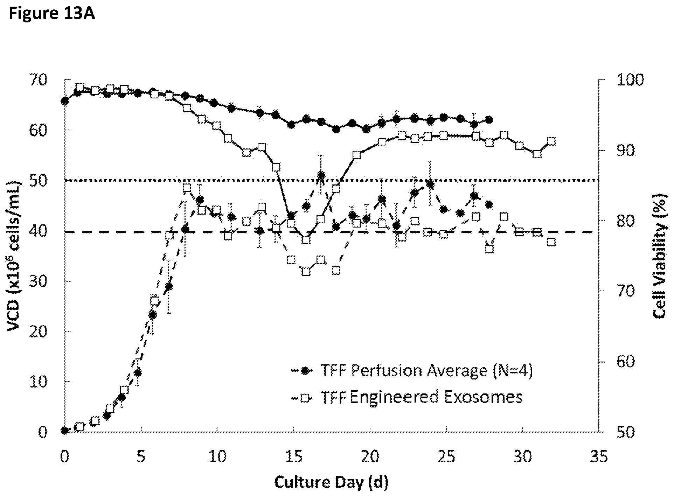

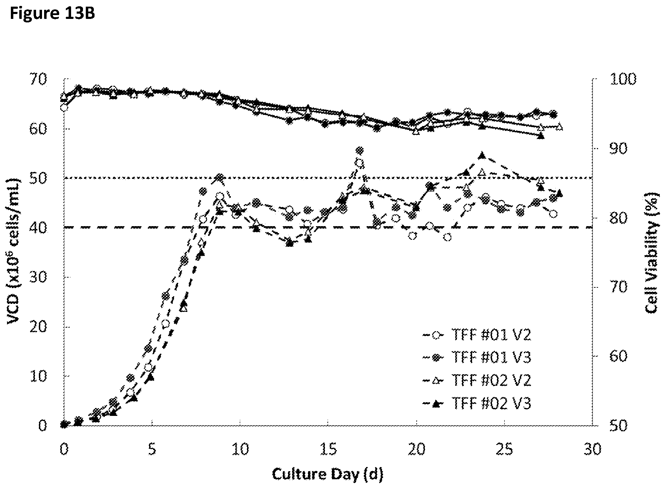

**Figure 13A** is a graph showing the average viable cell density (VCD; dotted lines) and cell viability (solid lines) over time for four different tangential flow filtration perfusion cell culture runs for wild-type cells and for one of PTGFRN-overexpressing cells. **Figure 13B** is a graph showing viable cell density (VCD; dotted lines) and cell viability (solid lines) over time for the four different tangential flow filtration perfusion cell culture runs for wild-type cells.

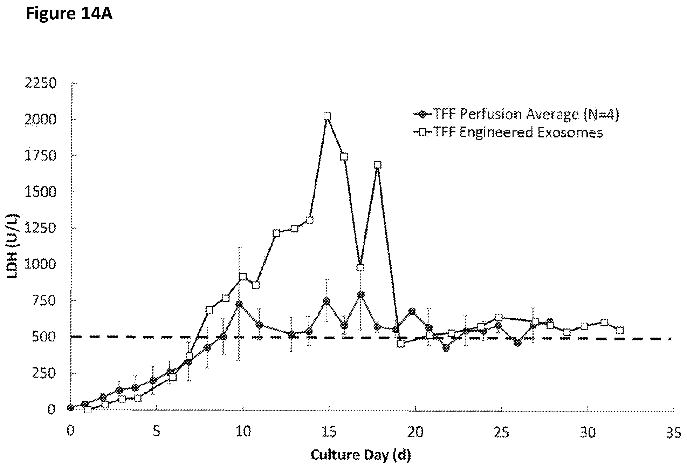

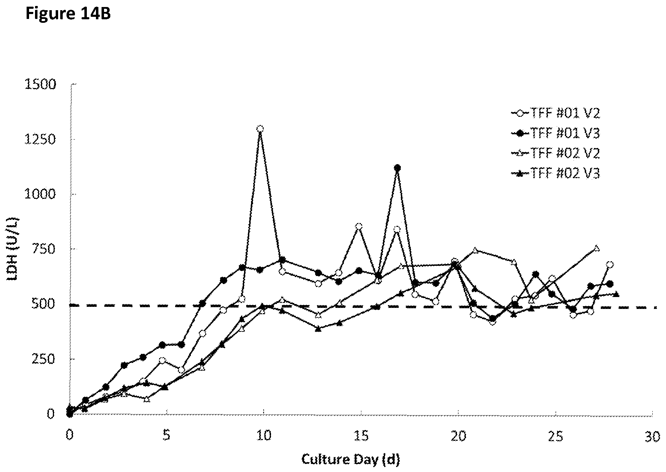

**Figure 14A** is a graph showing the average lactate dehydrogenase (LDH) levels over time for four different tangential flow filtration perfusion cell culture runs for wild-type cells and for one of PTGFRN-overexpressing cells. **Figure 14B** is a graph showing lactate dehydrogenase (LDH) levels over time for the four different tangential flow filtration perfusion cell culture runs for wild-type cells.

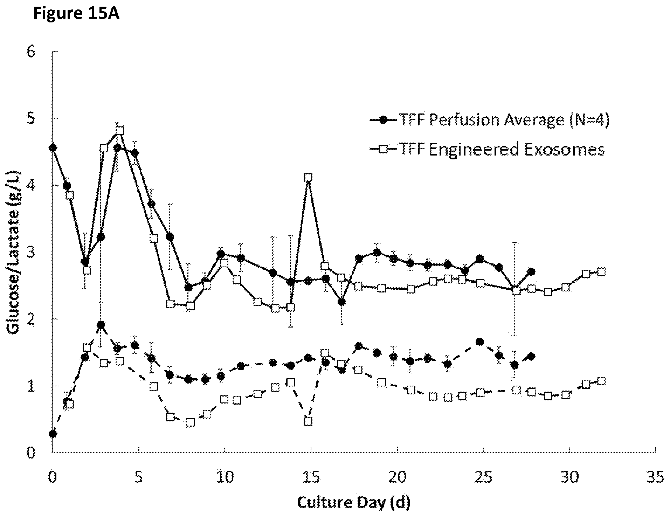

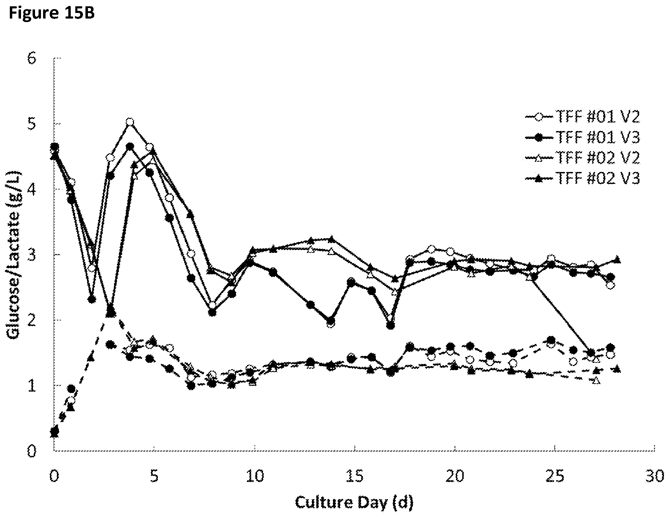

**Figure 15A** is a graph showing the average glucose (solid lines) and lactate (dotted lines) levels over time for four different tangential flow filtration perfusion cell culture runs for wild-type cells and for one of PTGFRN-overexpressing cells. **Figure 15B** is a graph showing glucose (solid lines) and lactate levels (dotted lines) over time for the four different tangential flow filtration perfusion cell culture runs for wild-type cells.

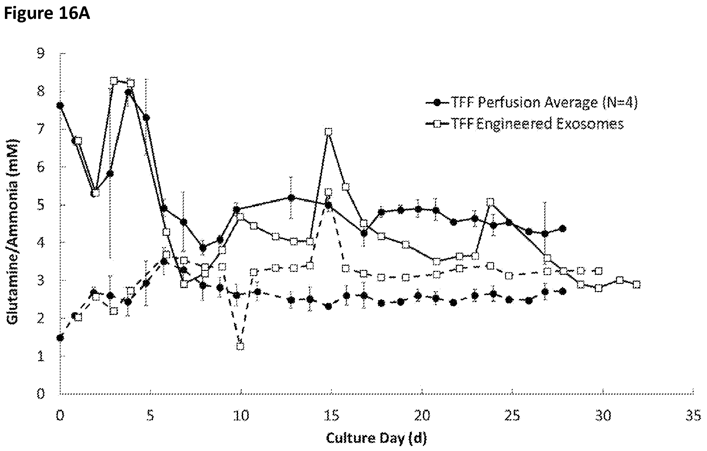

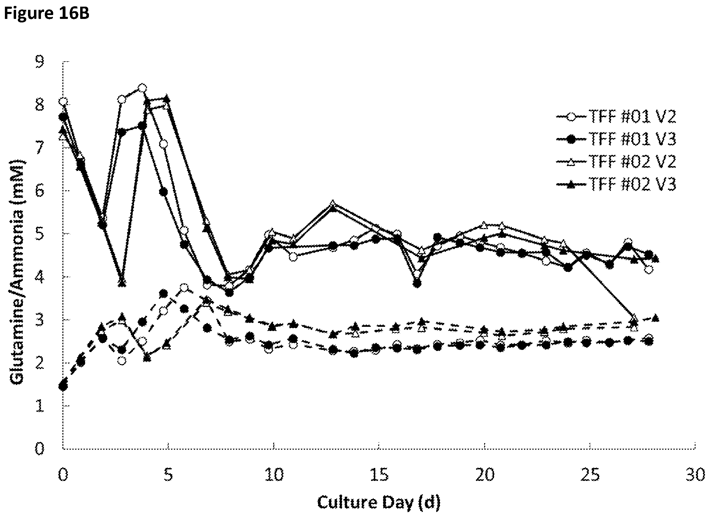

**Figure 16A** is a graph showing the average glutamine (solid lines) and ammonia (dotted lines) levels over time for four different tangential flow filtration perfusion cell culture runs for wild-type cells and for one of PTGFRN-overexpressing cells. **Figure 16B** is a graph showing glutamine (solid lines) and ammonia (dotted lines) levels over time for the four different tangential flow filtration perfusion cell culture runs for wild-type cells.

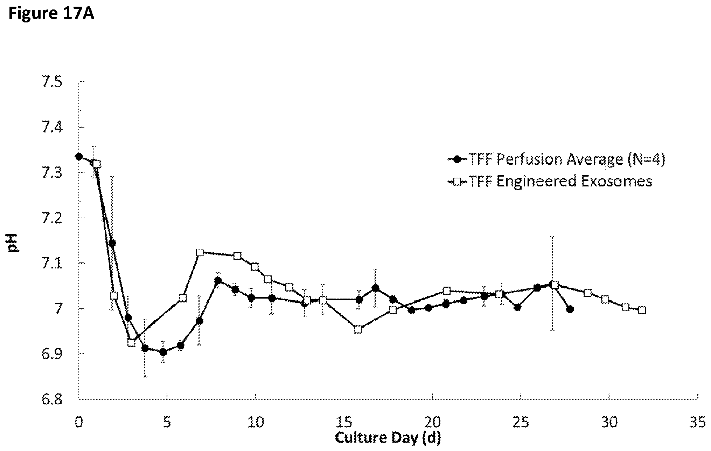

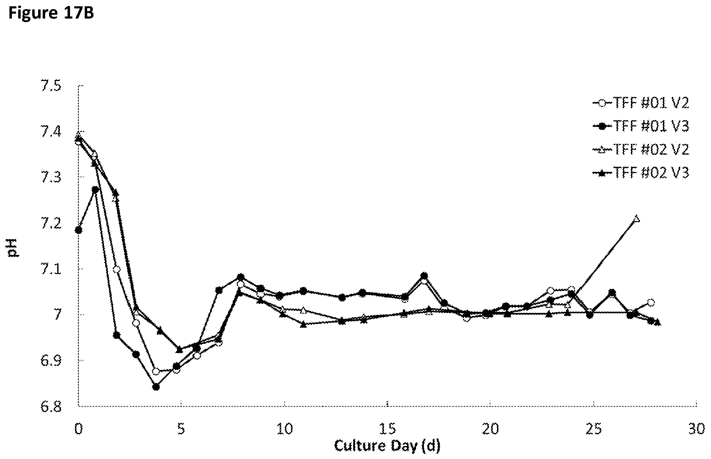

**Figure 17A** is a graph showing the average pH over time for four different tangential flow filtration perfusion cell culture runs for wild-type cells and for one of PTGFRN-overexpressing cells. **Figure 17B** is a graph showing pH over time for the four different tangential flow filtration perfusion cell culture runs for wild-type cells.

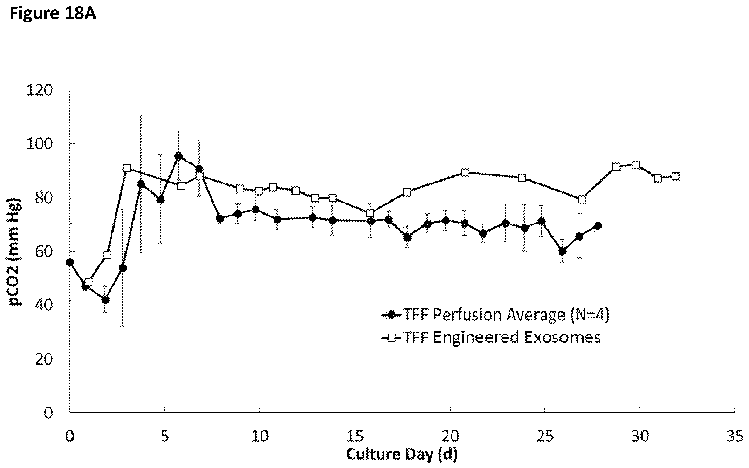

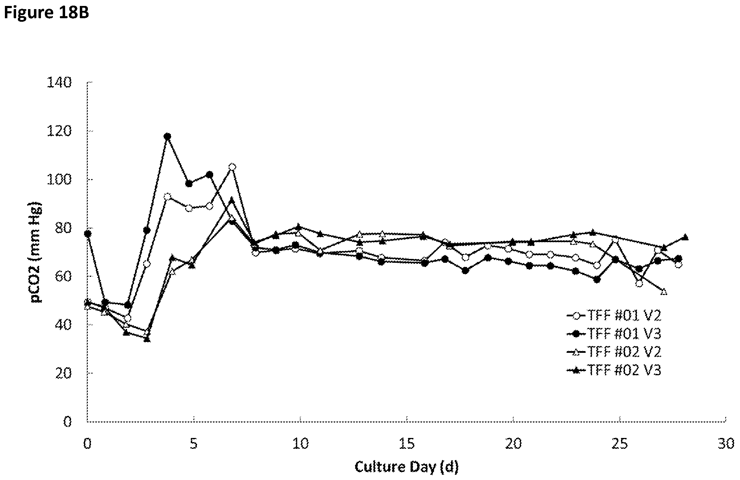

**Figure 18A** is a graph showing the average pCO₂ over time for four different tangential flow filtration perfusion cell culture runs for wild-type cells and for one of PTGFRN-overexpressing cells. **Figure 18B** is a graph showing pCO₂ over time for the four different tangential flow filtration perfusion cell culture runs for wild-type cells.

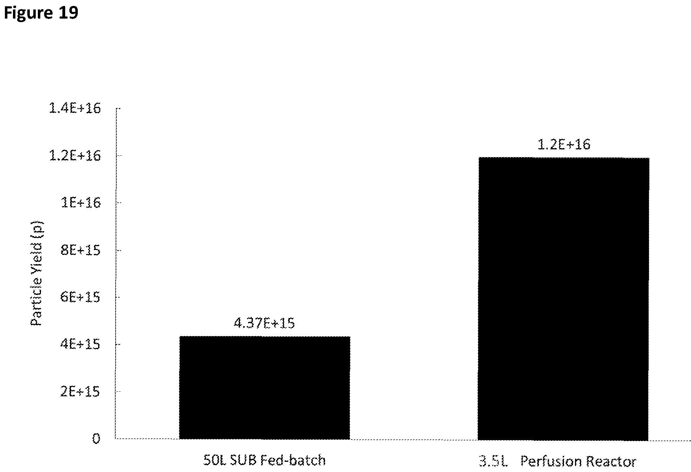

**Figure 19** is a chart showing total EV titer for a tangential flow filtration perfusion cell culture run compared to a 50L fed batch bioreactor run.

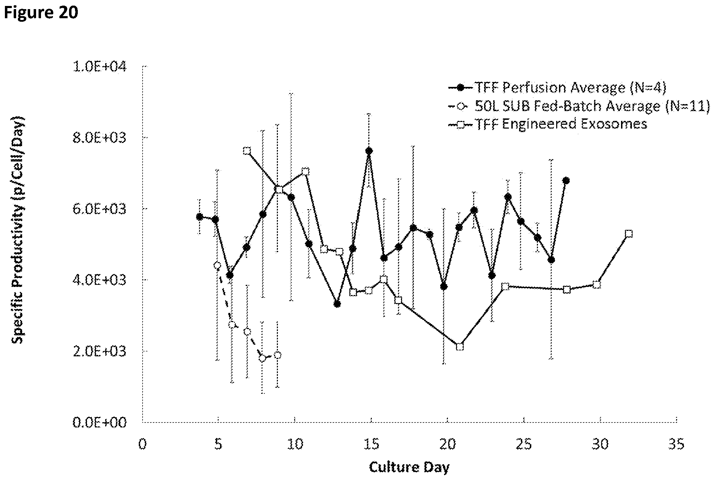

**Figure 20** is a graph showing the average EV specific productivity over time for four different tangential flow filtration perfusion cell culture runs for wild-type cells and for one of PTGFRN-overexpressing cells compared to eleven independent 50L fed batch bioreactor runs.

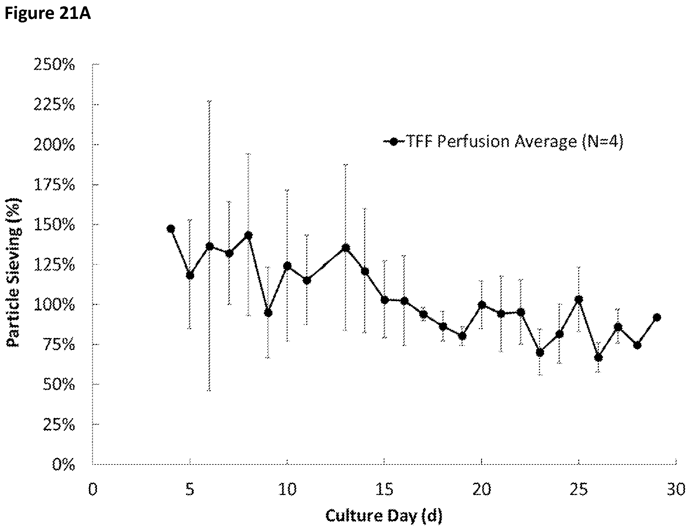

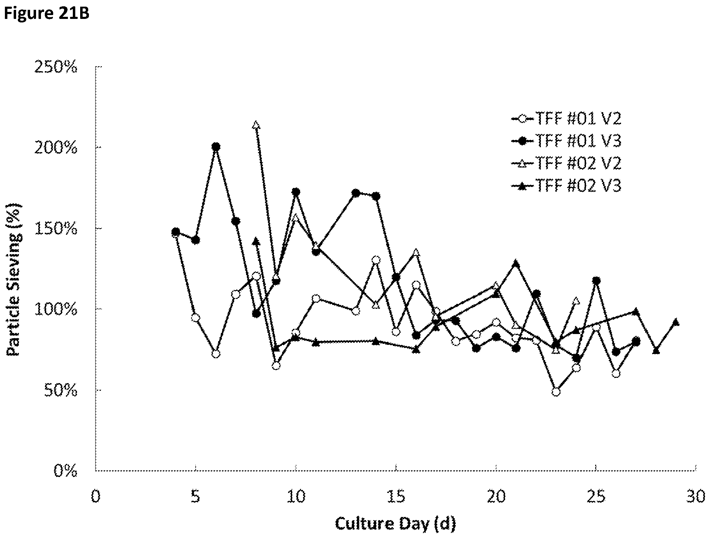

**Figure 21A** is a graph showing the average sieving coefficient of EVs across the tangential flow hollow fiber filter for four different tangential flow filtration perfusion cell culture runs. **Figure 21B** is a graph showing sieving coefficient of EVs across the tangential flow hollow fiber filter for the four different tangential flow filtration perfusion cell culture runs for wild-type cells.

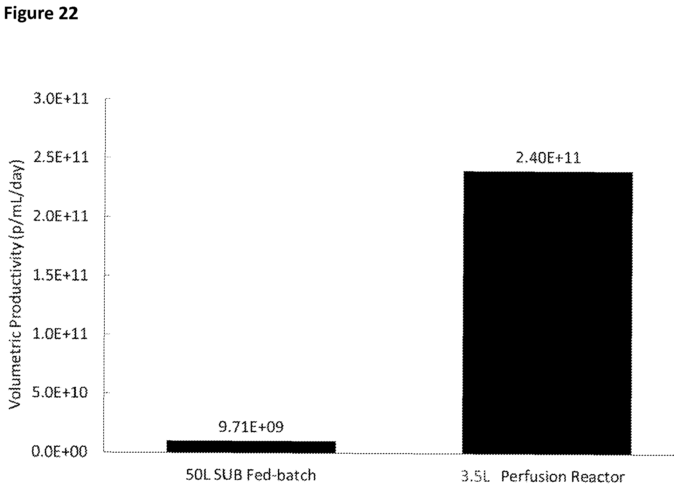

**Figure 22** is a graph comparing volumetric productivity from a 50L fed batch bioreactor run and a 3.5L perfusion reactor run.

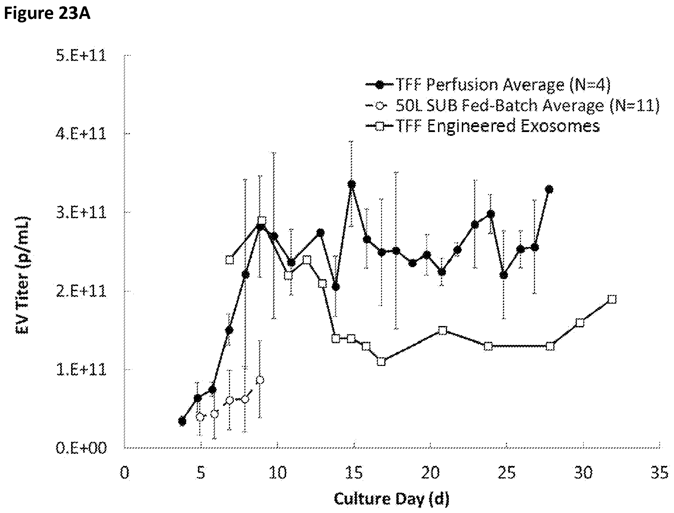

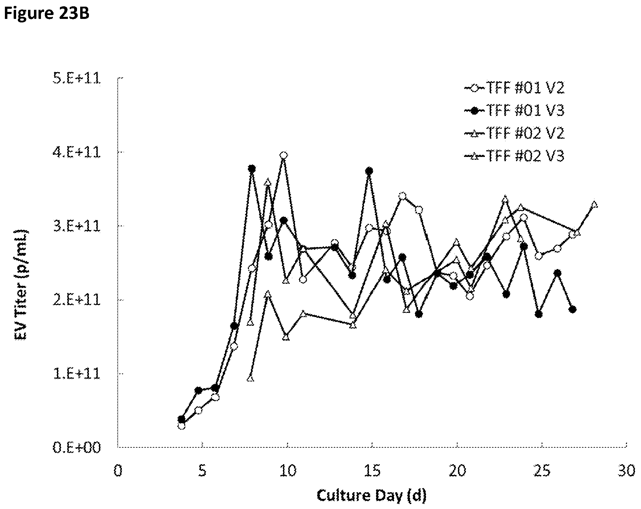

**Figure 23A** is a graph showing the average exosome titer over time for 11 independent 50L fed batch bioreactor runs compared to four independent 28 day 3.5L perfusion bioreactor runs for wild-type cells and one 28 day 3.5L bioreactor run for PTGFRN-overexpressing cells. Titer was measured by anion exchange HPLC with detection at 556 nm excitation, 573 nm emission. **Figure 23B** is a graph showing exosome titer over time for the four independent 28 day 3.5L perfusion bioreactor runs for wild-type cells.

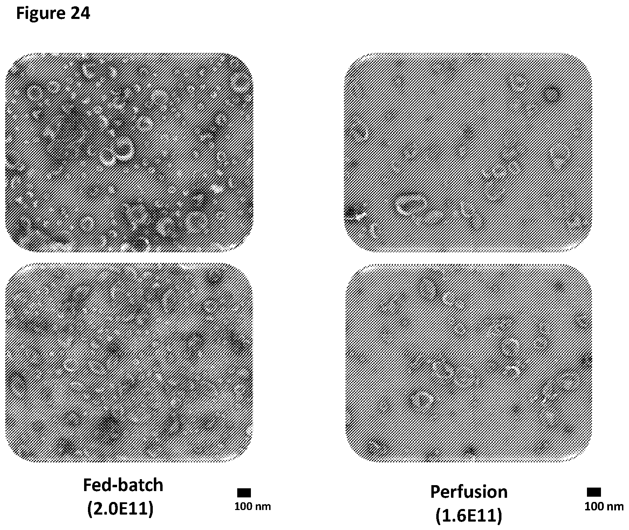

**Figure 24** is a series of electron micrographs of exosomes purified by Optiprep^{™} density-gradient ultracentrifugation from fed-batch and perfusion bioreactors.

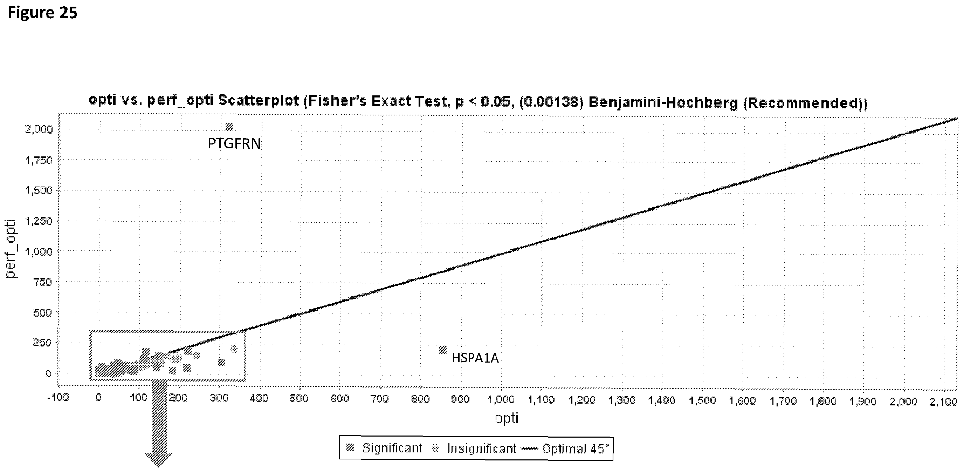

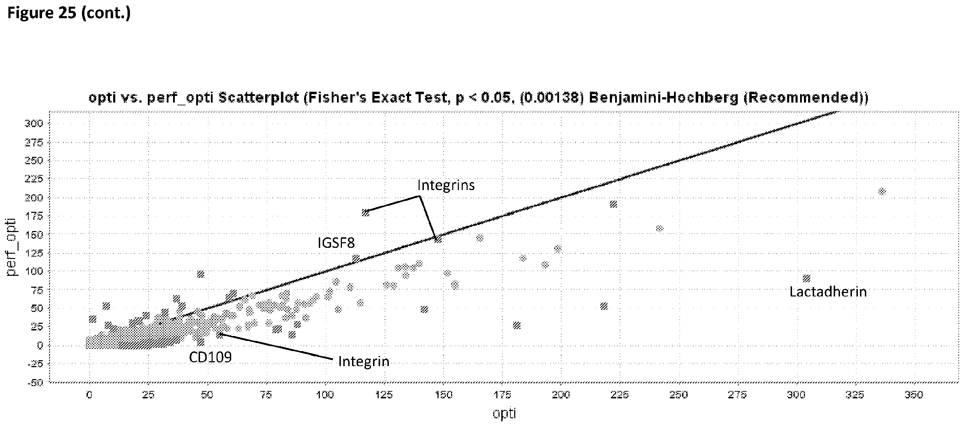

**Figure 25** is a scatterplot showing the identity and relative abundance of exosome-proteins from fed-batch and perfusion bioreactors.

### DETAILED DESCRIPTION

### Advantages and utility

Briefly, and as described in more detail below, are reliable methods for producing extracellular vesicles in chemically defined (CD) media in the absence of animal-derived components. The methods described herein allow for high titer production of allogenic exosomes in animal-derived component-free (ACF) suspension cell culture amenable to large scale manufacturing under cGMP conditions. Moreover, the developed processes described herein, is robust, and are compatible with single-use, disposable technology. The methods make exosome technology possible for a broad spectrum of therapies and large patient populations, which fulfills an unmet need in the field of exosome manufacturing, in particular for a broad clinical use. Thus, the methods described herein are a significant improvement over the state of the art and fulfill an unmet need in the field of extracellular vesicle manufacturing and quality control.

### Definitions

Terms used in the claims and specification are defined as set forth below unless otherwise specified.

"Chemically-defined media" refers to cell culture media that lack serum and any other animal-derived components and have known abundances of nutrients obtained in a controlled way through chemical, biochemical, or biological synthesis prior to addition of such media to cells.

"Batch fed," "fed-batch" or "fed batch" refers to cell culturing methods or cell cultures where cells remain in the culturing vessel until harvesting of the cells, in which an initial culture medium is added to an initial cell culture and additional feed medium is added to prevent nutrient depletion. In some embodiments, the feed medium is added once during the culturing process. In some embodiments, the feed medium is added multiple times during the culturing process. Fed batch culture includes constantly-fed batch culture and exponential-fed batch culture. Examples of fed batch culturing vessels include microwells, multitier plates, shallow well multitier plates, deep-well multitier plates, shake flasks, spin tubes, microbioreactors, bioreactors, such as the N-terminal production vessel, such as a stirred-tank bioreactor. In certain embodiments, the stirred-tank bioreactor is single use bioreactor, in certain embodiments the stirred tank bioreactor is a stainless-steel bioreactor, in certain embodiments, the stirred-tank bioreactor is a glass bioreactor.

"Batch re-feed," "batch-refeed" or "batch refeed" refers to cell culturing methods or cell cultures that closely approximate cell perfusion culturing methods and that do not include a cell-retention device. In certain embodiments, the batch refeed culture comprises use of a culturing vessel (e.g., deep-well multitier plates, shake flasks, spin tubes, microbioreactors) wherein cells are isolated for removal of spent medium and addition of fresh medium. In certain embodiments, the batch refeed culture comprises separating cells from spent medium by centrifugation. (*see* A. Villiger-Oberbek et al., 2015, J Biotechnol 212(10); 21-29).

"Perfusion culture" refers to cell culturing methods or cell cultures in which cells are continuously fed with fresh media and spent media is continuously removed while keeping cells in the culture vessel. In certain embodiments, culture vessels for perfusion culture comprise cell retention devices, such as capillary fibers or membranes. Examples of perfusion bioreactors with cell retention devices include bioreactors, such as the N-terminal production vessel, such as a stirred-tank bioreactor, connected to a cell retention device, such as hollow fiber filters or an acoustic cell separator. In certain embodiments, the perfusion culture is single-cell perfusion culture. In certain embodiments, the perfusion culture comprises use of a single-cell suspension perfusion bioreactor wherein individual cells are isolated for addition of fresh medium and removal of spent medium. In certain embodiments, the perfusion culture comprises separating cells from spent medium by centrifugation.

"Suspension culture" refers to cell culturing methods in which the cells do not adhere to a culture vessel substrate or other artificial substrate.

"Intrinsic fluorescence," "autofluorescence," and "auto-fluorescence" refer to the natural emission of light by biological structures after they have absorbed light, and is distinguished from light emitted by artificial fluorescent markers, dyes, or fluorophores. Intrinsic fluorescence of extracellular vesicles and exosomes is described in detail in International Patent Application PCT/US2017/066324, which is incorporated herein by reference in its entirety.

"Extracellular vesicle" refers to a cell-derived vesicle comprising a membrane that encloses an internal space. Extracellular vesicles comprise all membrane-bound vesicles that have a smaller diameter than the cell from which they are derived. Generally extracellular vesicles range in diameter from 20 nm to 1000 nm, and may comprise various macromolecular cargo either within the internal space, displayed on the external surface of the extracellular vesicle, and/or spanning the membrane. The cargo may comprise nucleic acids, proteins, carbohydrates, lipids, small molecules, and/or combinations thereof. By way of example and without limitation, extracellular vesicles include apoptotic bodies, fragments of cells, vesicles derived from cells by direct or indirect manipulation (e.g., by serial extrusion or treatment with alkaline solutions), vesiculated organelles, and vesicles produced by living cells (e.g., by direct plasma membrane budding or fusion of the late endosome with the plasma membrane). Extracellular vesicles may be derived from a living or dead organism, explanted tissues or organs, and/or cultured cells. Both nanovesicles and exosomes are species of extracellular vesicles.

"Nanovesicle" refers to a cell-derived small (ranging from 20-250 nm in diameter, more preferably 30-150 nm in diameter) vesicle comprising a membrane that encloses an internal space, and which is generated from the cell by direct or indirect manipulation such that the nanovesicle would not be produced by the producer cell without the manipulation. Appropriate manipulations of the producer cell include but are not limited to serial extrusion, treatment with alkaline solutions, sonication, or combinations thereof. The production of nanovesicles may, in some instances, result in the destruction of the producer cell. Preferably, populations of nanovesicles are substantially free of vesicles that are derived from producer cells by way of direct budding from the plasma membrane or fusion of the late endosome with the plasma membrane. The nanovesicle comprises lipid or fatty acid and polypeptide, and optionally comprises a payload *(e.g.,* a therapeutic agent), a receiver *(e.g.,* a targeting moiety), a polynucleotide (e.g., a nucleic acid, RNA, or DNA), a sugar *(e.g.,* a simple sugar, polysaccharide, or glycan) or other molecules. The nanovesicle, once it is derived from a producer cell according to the manipulation, may be isolated from the producer cell based on its size, density, biochemical parameters, or a combination thereof. Nanovesicles are a species of extracellular vesicles.

"Exosome" refers to a cell-derived small (ranging from 20-300 nm in diameter, more preferably 40-200 nm in diameter) vesicle comprising a membrane that encloses an internal space, and which is generated from the cell by direct plasma membrane budding or by fusion of the late endosome with the plasma membrane. Generally, production of exosomes does not result in the destruction of the producer cell. The exosome comprises lipid or fatty acid and polypeptide, and optionally comprises a payload *(e.g.,* a therapeutic agent), a receiver *(e.g.,* a targeting moiety), a polynucleotide (e.g., a nucleic acid, RNA, or DNA), a sugar *(e.g.,* a simple sugar, polysaccharide, or glycan) or other molecules. The exosome can be derived from a producer cell, and isolated from the producer cell based on its size, density, biochemical parameters, or a combination thereof. Exosomes are a species of extracellular vesicles.

"Parent cell" or "producer cell" include any cell from which an extracellular vesicle can be isolated. The terms also encompass a cell that shares a protein, lipid, sugar, or nucleic acid component of the extracellular vesicle. For example, a "parent cell" or "producer cell" may include a cell which serves as a source for the extracellular vesicle membrane.

"Purify," "purified," and "purifying" or "isolate," "isolated," or "isolating" or "enrich," "enriched" or "enriching" are used interchangeably and refer to the state of a population (e.g., a plurality of known or unknown amount and/or concentration) of desired extracellular vesicles, that have undergone one or more processes of purification, e.g., a selection or an enrichment of the desired extracellular vesicles composition, or alternatively a removal or reduction of residual biological products as described herein. In some embodiments, a purified extracellular vesicles composition has no detectable undesired activity or, alternatively, the level or amount of the undesired activity is at or below an acceptable level or amount. In other embodiments, a purified extracellular vesicle composition has an amount and/or concentration of desired extracellular vesicles at or above an acceptable amount and/or concentration. In other embodiments, the purified extracellular vesicle composition is enriched as compared to the starting material (e.g., biological material collected from tissue, bodily fluid, or cell preparations) from which the composition is obtained. This enrichment may be by 10%, 20%, 30%, 40%, 50%, 60%, 70%, 80%, 90%, 95%, 96%, 97%, 98%, 99%, 99.9%, 99.99%, 99.999%, 99.9999%, or greater than 99.9999% as compared to the starting material.

Abbreviations used in this application include the following: chemically-defined media (CD), fed-batch culturing (FB), batch re-feed culturing (BR), extracellular vesicles (EV or EVs), size-exclusion chromatography (SEC), nanoparticle tracking analysis (NTA), resistive pulse sensing (RPS), phosphate-buffered saline (PBS) and fluorescent activated cell sorting (FACS).

It must be noted that, as used in the specification and the appended claims, the singular forms "a," "an" and "the" include plural referents unless the context clearly dictates otherwise.

### Methods

### Sources of Extracellular Vesicles

Described herein are methods for producing extracellular vesicles from cells cultured in chemically defined (CD) media in the absence of animal-derived components. Extracellular vesicles can be produced by any type of cell from any organism. In certain embodiments, extracellular vesicles are produced by mammalian cells. In certain embodiments, extracellular vesicles are produced from commercially relevant cell lines. In certain embodiments, extracellular vesicles are produced from primary cells. In certain embodiments, extracellular vesicles are produced by human cells. In certain embodiments, extracellular vesicles are produced from Chinese hamster ovary (CHO) cells. In certain embodiments, the extracellular vesicles are produced from human mesenchymal stem cells. In certain embodiments, extracellular vesicles are produced from human kindney cells. In certain embodiments, extracellular vesicles are produced from HEK 293 SF cells. In certain embodiments, extracellular vesicles are produced by insect cells. In certain embodiments, extracellular vesicles are produced by yeast cells. In certain embodiments, extracellular vesicles are produced by bacterial cells. In certain embodiments, the cells are engineered cells. In certain embodiments, the engineered cells contain an exogenous nucleic acid sequence. In certain embodiments, said exogenous nucleic acid sequence encodes a peptide or a protein. In certain embodiments, said exogenous nucleic acid sequence is noncoding. In certain embodiments, said exogenous nucleic acid results in the deletion or modification of one or more endogenous DNA sequences. In certain embodiments, the engineered cells are generated by transfection of a DNA or RNA. In certain embodiments, the engineered cells are generated by viral transduction. In certain embodiments, the engineered cells are engineered by a genome editing complex, e.g., a CRISPR complex.

In certain embodiments, the cells producing extracellular vesicles are engineered to overexpress one or more exosome-specific proteins, thereby generating extracellular vesicles overexpressing said one or more exosome-specific proteins. In certain embodiments, said one or more exosome-specific proteins is a surface protein. In certain embodiments, said one or more exosome-specific proteins is a luminal protein. In certain embodiments, said one or more exosome-specific proteins is one or more luminal protein and one or more surface protein. In some embodiments, said surface protein is PTGFRN, BSG, IGSF2, IGSF3, IGSF8, ITGB1, ITGA4, SLC3A2, ATP transporter or a fragment or a variant thereof. In some embodiments, said surface proteins is PTGFRN or a fragment or a variant thereof. Exosome-specific surface proteins are described in detail in International Patent Application PCT/US2018/048026, which is incorporated herein by reference in its entirety. In some embodiments, said luminal protein is MARCKS, MARCKSL1, and/or BASP1, or a fragment or a variant thereof. In some embodiments, said luminal protein is BASP1 or a fragment or a variant thereof. Exosome-specific luminal proteins are described in detail in U.S. Patent Application 62/634,750, which is incorporated herein by reference in its entirety.

### Methods of Culturing Parent Cells for Production of Extracellular Vesicles

The methods disclosed herein comprise culturing cells for the production and isolation of extracellular vesicles. Cultured parent cells can be scaled up from high-throughput to bench-top scale to pilot bioreactor scale to manufacturing scale. For example, the parent cells are cultured until they reach saturation density, e.g., 1×10⁵, 1×10⁶, 1×10⁷, or greater than 1×10⁷ cells per ml. Optionally, upon reaching saturation density, the parent cells can be transferred to a larger volume of fresh medium.

In certain embodiments, exosomes isolated from the methods disclosed herein are harvested at high cell culture viabilities. In certain embodiments, the cell viability is 50% and above, 55%, 60%, 65%, 75%, 80%, 85%, 90% or 95% and above.

In certain embodiments, exosomes isolated from the methods disclosed herein have reduced microvesicular and other membranous contamination, thus simplifying the downstream operations associated with the developed upstream exosome production process.

In certain embodiments, exosomes isolated from the methods disclosed herein have reduced protein, nucleic acid and other cell contaminants.

### Cell Culture Media

Unlike the more common method of producing exosomes in adherent cultures often containing animal-derived components, the application discloses use of suspension culture and chemically-defined (CD) media from vial thaw, through seed expansion, scale-up, and growth and production.

Any suitable CD medium can be used for culturing and expansion of the parent cells of interest that supports the growth of the cells in suspension culture. Examples of CD media that can be used that are commercially available include, but are not limited to: CD 293 Medium (ThermoFisher Scientific Catalogue number #11913-019), FreeStyle^{™} 293 Expression Medium (ThermoFisher Scientific Catalogue number #12338), EfficientFeed^{™} A (ThermoFisher Scientific Catalogue number #A1023401), EfficientFeed^{™} B (ThermoFisher Scientific Catalogue number #A1024401), EfficientFeed^{™} C (ThermoFisher Scientific Catalogue number #A2503101), EX-Cell 293 Serum-Free Medium (SAFC, catalogue #14571C), EX-Cell CHOZN Platform Feed (SAFC, Catalogue #24331C), EX-Cell Advanced^{™} CHO Feed 1 (SAFC, Catalogue #24368C), HyClone^{™}CDM4HEK293 (GE Healthcare, Catalogue #SH30858), ), HyClone^{™}CDM4PERMAb (GE Healthcare, Catalogue #SH30871), ), HyClone^{™}SFM4Transfx-293 (GE Healthcare, Catalogue #SH30860), HyClone^{™} Cell Boost^{™} 1(GE Healthcare, Catalogue #SH30584), HyClone^{™} Cell Boost^{™}2 (GE Healthcare, Catalogue #SH30596), HyClone^{™} Cell Boost^{™} 3(GE Healthcare, Catalogue #SH30825), HyClone^{™} Cell Boost^{™} 4(GE Healthcare, Catalogue #SH30857), HyClone^{™} Cell Boost^{™} 5(GE Healthcare, Catalogue #SH30865), HyClone^{™} Cell Boost^{™}6 (GE Healthcare, Catalogue #SH30866), Xell HEK GM (Xell AG, Catalogue #851), Xell HEK TF (Xell AG, Catalogue #861), and Xell HEK SF (Xell AG, Catalogue #871).

### Culturing Conditions

Culturing conditions will vary according to the culturing method employed (i.e., fed-batch, batch-refeed, or perfusion) to obtain adequate cell densities, cell viability and reduced contaminants. Agitation conditions for culturing to ensure proper aeration at sufficiently high cell densities can vary and depend upon cell type, type of culture vessel, volume of culture, etc. Agitation can be achieved by stirring or shaking. In certain embodiments, the speed of shaking ranges from 350 rpm and 10 rpm, or ranges from 300 rpm and 50 rpm, or ranges from 275 and 150 rpm, or ranges from 250 and 200 rpm. In certain embodiments, the speed of shaking is 250 rpm. In certain embodiments, the speed of agitation will vary according to cell culture volume, shaking diameter and/ or if the culture vessel is tilted or cultured at 90 degree angle. In certain embodiments, the culture vessel is agitated at a 90 degree angle. In certain embodiments, the culture vessel is agitated at a 45 degree angle. In certain embodiments, the culture vessel is agitated at an angle ranging from a 5 degree angle and a 90 degree angle. In certain embodiments, the culture vessel has a diameter ranging from 15 mm and 75 mm. In certain embodiments, the culture vessel has a diameter ranging from 25 mm and 50 mm. In certain embodiments, the culture vessel has a diameter of 25 mm. In certain embodiments, the culture vessel has a diameter of 50 mm. In certain embodiments, the culturing condition comprises a 50 mm culture vessel stirring or shaking at a speed of 180 rpm at an agitation angle of 90 degrees. In certain embodiments, the culturing condition comprises a 25 mm culture vessel stirring or shaking at a speed of 160 rpm at an agitation angle of 45 degrees. In certain embodiments, the culturing condition comprises a 25 mm culture vessel stirring or shaking at a speed of 250 rpm at an agitation angle of 90 degrees. In certain embodiments, the culturing condition comprises a 25 mm culture vessel stirring or shaking at a speed of 220 rpm at an agitation angle of 90 degrees.

Culture volume will also vary according to cell culturing methods and cell type. In certain embodiments, the cells are cultured at a volume ranging from 0.1 mL and 20 L. In certain embodiments, the cells are cultured at a volume ranging from 0.1 mL and 1 mL, ranging from 1 mL and 10 mL, ranging from 10 mL and 100 mL, ranging from 100 mL and 1 L, ranging from 1 L and 10 L, or ranging from 10 L and 50 L.

The number of days of culture prior to cell and/or exosome harvest will depend on cell type, culture conditions, etc. In certain embodiments, the cells are cultured for a duration ranging from 3 days and 21 days prior to harvest. In certain embodiments, the cells are cultured 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, or 22 days to 30 days, or more prior to harvest.

In certain embodiments, the cells are supplied with fresh medium intermittently. In certain embodiments, the cells are supplied with fresh medium continuously. In certain embodiments, the cells are supplied with fresh medium intermittently, every 24 h. In certain embodiments, the cells are supplied with fresh medium intermittently, at a duration ranging from every 1 h to every 48 h, ranging from every 1 h to every 2 h, ranging from every 2 h to every 6 h, ranging from every 6 h to every 12 h, ranging from every 12 h and every 24 h, or ranging from every 24 h and every 48 h.

### Culture Vessels

The type of cell culture vessel used will depend upon they type of culturing methods employed and/or bioreactor (e.g., a perfusion bioreactor with a cell retention device or a batch-refeed method with no retention device, etc.). Any suitable cell culture vessel may be used for suspension culture including, but not limited to, bioreactors, shake flasks, Tubespin^{™} bioreactors, shake tubes, and microtiter plates. Non-limiting examples of cell culture vessels include conical tubes, shallow-well multititer plates, deep-well multititer plates, microbioreactors, glass bioreactors, stainless steel bioreactors, or single use bioreactors. The size of the culture vessel can be of any size suitable for the culture method and/or volume of cell culture to achieve optimal cell densities and viability with minimal contaminants.

### Batch Re-Feed Culturing

In certain embodiments, the culturing method is a batch re-feed method. In certain embodiments, the batch re-feed method comprises use of shake tubes. In certain embodiments, the shake tubes are agitated at a shake speed of 160 rpm. In certain embodiments, the shake tubes are agitated at a shake speed ranging from 350 rpm and 10 rpm, or ranging from 300 rpm and 100 rpm, or ranging from 250 and 150 rpm, or ranging from 250 and 200 rpm.

In certain embodiments, the culture vessel is agitated at a 90 degree angle. In certain embodiments, the culture vessel is agitated at a 45 degree angle. In certain embodiments, the culture vessel is agitated at a range of a 5 degree angle to a 90 degree angle.

Culture volume will also vary according to cell culturing methods and cell type. In certain embodiments, the cells are cultured at a volume ranging from 0.1 mL and 20 L. In certain embodiments, the cells are cultured at a volume ranging from 0.1 mL and 1 mL, ranging from 1 mL and 10 mL, ranging from 10 mL and 100 mL, ranging from 100 mL and 1 L, ranging from 1 L and 10 L, ranging from 10 L and 50 L, or 50 L and greater.

In certain embodiments, the batch re-feed method results in increased cell viability compared to cells cultured using a fed-batch culturing method cultured for the same number of days. In certain embodiments, the increased cell viability is two-fold or greater cell viability after 9 days of culture. In certain embodiments, the extracellular vesicle preparation from batch re-feed cultures comprises reduced proteinaceous contaminants, nucleic acid contaminants, small molecule, metabolites, membranous contaminants, or combinations thereof, compared to extracellular vesicle preparations harvested using a fed-batch culturing method cultured for the same number of days. In certain embodiments, the reduced proteinaceous contaminants, small molecules, metabolites, membranous contaminants, nucleic acid contaminants or combinations thereof are less than 60% for first 10 days of culture.

In certain embodiments, the extracellular vesicle preparation from batch re-feed cultures comprises increased abundance of extracellular vesicles, compared to extracellular vesicle preparations harvested from cultures using a fed-batch culturing method that are cultured for the same number of days. In certain embodiments, the increased abundance of extracellular vesicles is greater than 2-fold after 9 days of culture. In certain embodiments, the increased abundance of extracellular vesicles is greater than 3-fold to 10-fold after 10 days of culture. In certain embodiments, the volumetric productivity of extracellular vesicles from batch-refeed cultures is greater than 2 × 10¹⁰ extracellular vesicle particles/ml culture/day. In certain embodiments, the volumetric productivity of extracellular vesicles from batch-refeed cultures ranges from 1 × 10¹⁰ and 3 × 10¹⁰extracellular vesicle particles/ml culture /day. In certain embodiments, the extracellular vesicle preparation comprises exosomes. In certain embodiments, the extracellular vesicle preparation comprises exosomes and other extracellular vesicle particles.

### Perfusion Culturing

In certain embodiments, single-cell perfusion is performed. Single cell perfusion allows for a decreased bioreactor footprint, higher cell density, higher exosome yield and further improved purity of the exosome harvest over extended period, as compared to fed-batch, adding another benefit of decreased manufacturing costs and yet more streamlined downstream processing.

In certain embodiments, culture vessels for perfusion culture comprise cell retention devices, such as capillary fibers (e.g., hollow fibers) or membranes. In certain embodiments, said hollow fibers have a pore size of 0.45 µm, 0.5 µm, 0.65 µm, 0.8 µm 1-3 µm, or 2-5 µm. In certain embodiments, the perfusion culture is single-cell perfusion culture. In certain embodiments, the perfusion culture comprises use of a single-cell perfusion bioreactor wherein individual cells are isolated for addition of fresh medium and removal of spent medium. In certain embodiments, the perfusion culture comprises separating from spent medium by centrifugation. In certain embodiments, the perfusion culture comprises separating from spent medium by one or more filters. In certain embodiments, the perfusion culture is tangential flow filtration perfusion. In certain embodiments, the perfusion culture is alternating tangential flow filtration perfusion.

In certain embodiments, the perfusion culturing method results in increased cell viability compared to cells cultured using a fed-batch culturing method cultured for the same number of days. In certain embodiments, the increased cell viability is two-fold or greater cell viability after 10 days of culture. In certain embodiments, the extracellular vesicle preparation from perfusion cultures comprises reduced proteinaceous contaminants, nucleic acid contaminants, small molecule, metabolites, membranous contaminants, or combinations thereof, compared to extracellular vesicle preparations harvested using a fed-batch culturing method cultured for the same number of days. In certain embodiments, the reduced proteinaceous contaminants, small molecules, metabolites, membranous contaminants, nucleic acid contaminants or combinations thereof are less than 60% for first 10 days of culture.

In certain embodiments, the extracellular vesicle preparation from perfusion cultures comprises increased abundance of extracellular vesicles, compared to extracellular vesicle preparations harvested from cultures using a fed-batch culturing method that are cultured for the same number of days. In certain embodiments, the increased abundance of extracellular vesicles is greater than 2-fold after 10 days of culture. In certain embodiments, the increased abundance of extracellular vesicles is greater than 3-fold to 10-fold after 10 days of culture. In certain embodiments, the volumetric productivity of extracellular vesicles from perfusion cultures is greater than 1 × 10¹¹ extracellular vesicle particles/ml culture/day. In certain embodiments, the titer of extracellular vesicles from perfusion cultures is ranges from 1 × 10¹³ and 1 × 10¹⁴, ranges from 1 × 10¹⁴ and 1 × 10¹⁵, or ranges from 1 × 10¹⁵ and 1 × 10¹⁶ extracellular vesicle particles/ml culture/day. In certain embodiments, the yield of extracellular vesicles from perfusion cultures is greater than 3 × 10¹⁵ extracellular vesicle particles/2.4 L reactor volume/12 days.

In certain embodiments, the extracellular vesicle preparation comprises exosomes. In certain embodiments, the extracellular vesicle preparation comprises exosomes and other extracellular vesicle particles.

### Bioreactors

The parent cells may be cultured in a bioreactor, such as, e.g., a WAVE bioreactor, a stirred-tank bioreactor, a shaken bioreactor. Various configurations of bioreactors are known in the art and a suitable configuration may be chosen as desired. In certain embodiments, the culture is performed in, e.g., an N-1 or N-terminal vessel, or a bioreactor. In certain embodiments, the culture vessel is a fed-batch or perfusion bioreactor. In certain embodiments, a bioreactor is connected to a cell separator. For example, an N-terminal production vessel, in particular, a stirred-tank bioreactor, connected to a cell retention device, such as hollow fibrous membranes (run in, e.g., alternating tangential flow filtration (ATF), tangential flow filtration (TFF)), or an acoustic cell separator are used.

Configurations suitable for culturing and/or expanding populations of parent cells can easily be determined by one of skill in the art without undue experimentation. The bioreactor can be oxygenated. The bioreactor may optionally contain one or more impellers, a recycle stream, a media inlet stream, and control components to regulate the influx of media and nutrients or to regulate efflux of media, nutrients, and waste products.

### Cell Viability, Cell Titers and Yield of EVs

In certain embodiments, the viability of the cells after 7 days in culture is greater than 80%. In certain embodiments, the cell viability is greater than 85% after 7 days in culture. In certain embodiments, the cell viability ranges from 50% and 99.9%, ranges from 50% and 60%, ranges from 60% and 70%, ranges from 70% and 80%, ranges from 80% and 90 %, or ranges from 90% and 100% after 7 days in culture. In certain embodiments, the cell viability is reduced after 7 days of culture.

In certain embodiments cells are cultured to high cell densities, e.g., greater than 1×10⁴, 1×10⁵, 1×10⁶, 1×10⁷, 1×10⁸, 1×10⁹, or greater than 1×10⁹ cells per ml of culture.

In certain embodiments, the yield of extracellular vesicles after harvesting is greater than 3 × 10¹⁵ extracellular vesicle particles/2.4 L reactor volume/12 days. In certain embodiments, the yield of extracellular vesicles after harvesting is greater than 1 × 10⁹ extracellular vesicle particles/reactor volume/run duration, greater than 1 × 10¹⁰ extracellular vesicle particles/reactor volume/run duration, greater than 1 × 10¹¹ extracellular vesicle particles/reactor volume/run duration, greater than 1 × 10¹² extracellular vesicle particles/reactor volume/run duration, greater than 1 × 10¹³ extracellular vesicle particles/reactor volume/run duration, greater than 1 × 10¹⁴ extracellular vesicle particles/reactor volume/run duration, or greater than 1 × 10¹⁵ extracellular vesicle particles/reactor volume/run duration.

### Enrichment of Extracellular Vesicle Preparations

With respect to purification or enrichment of extracellular vesicles, it is contemplated that all known manners of purification of extracellular vesicles are deemed suitable for use herein. For example, physical properties of extracellular vesicles may be employed to separate them from a medium or other source material, including separation on the basis of electrical charge (e.g., electrophoretic separation, ion-exchange chromatography), size (*e.g.,* filtration, size-exclusion chromatography, molecular sieving, *etc*.), density (e.g., regular or gradient centrifugation), Svedberg constant (e.g., sedimentation with or without external force, *etc*.). For ion-exchange chromatography, any suitable methods known in the art may be used including, but not limited to, anion-exchange chromatography, and strong-anion exchange chromatography. For density gradient centrifugation, any appropriate density gradient medium used in the art may be used, including, but not limited to, sucrose density gradient medium and mediums comprising, iodixanol solution, colloidal silica, inorganic salts, polyhydric alcohols, polysaccharides, poly(vinyl alcohol), iohexol and nonionic iodinated media. Purification of the extracellular vesicles may be performed by manually loading columns or other devices, or may be automated using devices such an autosampler.

Alternatively, or additionally, isolation can be based on one or more biological properties, and include methods that can employ surface markers (e.g., precipitation, reversible binding to solid phase, FACS separation, separation using magnetic surfaces, specific ligand binding, immunoprecipitation or other antibody-mediated separation techniques, non-specific ligand binding such as annexin V, *etc*.). In yet further contemplated methods, the extracellular vesicles can also be fused using chemical and/or physical methods, including PEG-induced fusion and/or ultrasonic fusion.

In certain embodiments, enrichment of extracellular vesicles can be done in a general and non-selective manner (e.g., methods comprising serial centrifugation), and can be performed by aggregation where the extracellular vesicles are interlinked with an interlinking composition (e.g., annexin V, fibrin, or an antibody or fragment thereof against at least one of a tetraspanin, ICAM-1, CD86, CD63). Alternatively, enrichment of extracellular vesicles can be done in a more specific and selective manner (e.g., using tissue or cell specific surface markers). For example, specific surface markers can be used in immunoprecipitation, FACS sorting, bead-bound ligands for magnetic separation, *etc.*

In some embodiments, size exclusion chromatography can be utilized to enrich the extracellular vesicles. Size exclusion chromatography techniques are known in the art. Exemplary, non-limiting techniques are provided herein. In some embodiments, a void volume fraction is isolated and comprises extracellular vesicles of interest. Further, in some embodiments, the extracellular vesicles can be further isolated after chromatographic separation by centrifugation techniques (of one or more chromatography fractions), as is generally known in the art. In some embodiments, for example, density gradient centrifugation can be utilized to further enrich the extracellular vesicles. Still further, in some embodiments, it can be desirable to further separate the parent-cell derived extracellular vesicles from extracellular vesicles of other origin. For example, the parent cell extracellular vesicles can be separated from non-parent cell-derived extracellular vesicles by immunosorbent capture using an antigen antibody specific for the parent cell. For example, anti-CD63 antibodies can be used.

In some embodiments, the isolation of extracellular vesicles can involve combinations of methods that include, but are not limited to, differential centrifugation, size-based membrane filtration, concentration and/or rate zonal centrifugation, and further characterized using methods that include, but are not limited to, electron microscopy, flow cytometry and Western blotting.

Extracellular vesicles can be extracted from the supernatant of parent cells and demonstrate membrane and internal protein, lipid, and nucleic acid compositions that enable their efficient delivery to and interaction with recipient cells. Extracellular vesicles can be derived from parent cells that may include, but are not limited to, reticulocytes, erythrocytes, megakaryocytes, platelets, neutrophils, tumor cells, connective tissue cells, neural cells and stem cells. Suitable sources of extracellular vesicles include but are not limited to, cells isolated from subjects from patient-derived hematopoietic or erythroid progenitor cells, immortalized cell lines, or cells derived from induced pluripotent stem cells, optionally cultured and differentiated. Cell culture protocols can vary according to compositions of nutrients, growth factors, starting cell lines, culture period, and morphological traits by which the resulting cells are characterized. In some embodiments, the samples comprising extracellular vesicles are derived from a plurality of donor cell types (e.g., 2, 3, 4, 5, 6, 7, 8, 9, 10, 50, 100, 500, 1000, 5000, or 10000 donor cell types) and are combined or pooled. Pooling can occur by mixing cell populations prior to extracellular vesicles extraction or by mixing isolated extracellular vesicles compositions from subsets of donor cell types. Parent cells can be irradiated or otherwise treated to affect the production rate and/or composition pattern of secreted extracellular vesicles prior to isolation.

In certain embodiments, the extracellular vesicles can be derived from cell lines that are differentiated, proliferated and cultured *in vitro.* This enables controllable and reproducible compositions of extracellular vesicles that are not subject to constraints on isolation and purification of the requisite parent cell type.

In certain embodiments, the samples comprising the extracellular vesicles are obtained from raw cell harvest and the intrinsic fluorescence is determined. In certain embodiments, the raw cell harvest is clarified for larger cells and cellular debris prior to determination of the intrinsic fluorescence. In certain embodiments, the samples comprising the extracellular vesicles are further purified using any of the above mentioned methods for enrichment of the extracellular vesicles prior to determination of the intrinsic fluorescence of the samples.

In certain embodiments, the methods comprise fractionating the sample prior to determination of the intrinsic fluorescence. In certain embodiments, the method comprises the steps of loading the extracellular vesicle preparation on a size exclusion chromatography (SEC) column (e.g., a sepharose resin SEC column). In certain embodiments, the methods comprise the steps of loading the extracellular vesicle preparation on an ion exchange chromatography column. In certain embodiments, the methods comprise the steps of loading the extracellular vesicle preparation on a strong anion exchange chromatography column.

### Detection of Intrinsic Florescence

In certain embodiments, the intrinsic fluorescence of the eluted fractions from columns used for separation and/or fractionation of the samples comprising the extracellular vesicles is detected in a single step. In certain embodiments, the intrinsic fluorescence of the eluted fractions from the columns used for separation and/or fractionation is detected in multiple steps. In certain embodiments, the detection of intrinsic fluorescence of the eluted fractions is detected after the fractions have been further processed or stored for a period of time. In certain embodiments, the eluted fractions are analyzed for intrinsic fluorescence on the same device as the device used for separation and/or fractionation of the sample comprising the extracellular vesicles. In certain embodiments, the fractions are analyzed for intrinsic fluorescence on a separate device as the device used for separation and/or fractionation of the sample comprising the extracellular vesicles. In certain embodiments, the sample fractions are collected using a flow-cell.

The relative amounts or concentrations of extracellular vesicle are determined by measurement of intrinsic fluorescence. Detection and/or measurement of intrinsic fluorescence can be performed manually by fluorescent microscopy or determined using automated systems for fluorescent detection. In certain embodiments, intrinsic fluorescence of the extracellular vesicle preparations or fractions of extracellular vesicle preparation is determined using a microplate reader or any other acceptable method known in the art for the detection and measurement of fluorescence in a sample.

In certain embodiments, extracellular vesicle preparation can be sorted by flow cytometry, *e.g.,* bead-based flow cytometry as described in Melo et al. (Nature, 2015 Jul 9;523[7559]: 177-82) based on intrinsic fluorescence at particular excitation and emission spectra.

In certain embodiments, the intrinsic fluorescence profile of extracellular vesicles is detected at a range of 450 to 550 nm absorbance wavelength and at a range of 470 to 570nm emission wavelength. In certain embodiments, the intrinsic fluorescence profile of extracellular vesicles is detected at a range of 450 to 460 nm, 460 to 470 nm, 470 to 480 nm, 480 to 490 nm, 490 to 500 nm, 500 to 510 nm, 510 to 520 nm, 520 to 530 nm and 540 to 550 nm absorbance wavelength. In certain embodiments, the intrinsic fluorescence profile of extracellular vesicles is detected at a range of 470 to 480 nm, 480 to 490 nm, 490 to 500 nm, 500 to 510 nm, 520 to 530 nm, 530 to 540 nm, 540 to 550 nm, 550 to 560 nm and 560 to 570 nm emission wavelength, respectively. In an aspect, the intrinsic fluorescence emission signal is generated using an excitation wavelength ranging from 450 nm to 650 nm and an emission wavelength that is longer than the excitation wavelength and ranging from 470 nm to 670 nm. In an aspect, the intrinsic fluorescence emission signal is determined at an emission wavelength range of 500 to 600 nm. In an aspect, the intrinsic fluorescence emission signal is determined at an emission wavelength range of 550 to 590 nm. In an aspect, the intrinsic fluorescence emission signal is determined at an emission wavelength of 573 nm. In an aspect, the intrinsic fluorescence emission signal is determined at an excitation wavelength range of 500 to 600 nm. In an aspect, the intrinsic fluorescence emission signal is determined at an excitation wavelength range of 530 to 570 nm. In an aspect, the intrinsic fluorescence emission signal is determined at an excitation wavelength of 556 nm. In an aspect, the intrinsic fluorescence emission signal is determined at an excitation wavelength of 550 nm and an emission of 570 nm. But, as is well recognized by those of skill in the art, in each instance, the absorbance wavelength is shorter than the emission wavelength.

In certain aspects, the absorbance wavelength varies according to the membrane composition and/or payload composition of the extracellular vesicle. In certain aspects, the emission wavelength varies according to the membrane composition and/or payload composition of the extracellular vesicle. In certain aspects, the emission wavelength and/or absorbance wavelength varies according to the homogeneity of the extracellular vesicle preparation. In certain aspects, the absorbance wavelength and/or emission wavelength used to detect the extracellular vesicle varies according to the type of producer cell from which the extracellular vesicle is derived. In certain aspects, the absorbance wavelength and/or emission wavelength used to detect the extracellular vesicle varies according to the purity of the extracellular preparation prior to detection of the extracellular vesicles.

### Quantitation of Concentration of Extracellular Vesicle Preparations

In certain aspects, the methods comprise displaying the absorbance and/or emission spectra obtained from the sample on a chromatogram. In certain aspects, the relative amounts or concentrations of extracellular vesicles in the sample or a fraction of the sample is obtained by calculating the area under the resulting absorbance curve of the chromatogram and calculating the same using a quantification standard, wherein the standard is applied to a similar extracellular vesicle preparation. The concentration of the extracellular vesicles in the quantification standard can be measured by any of the known methods in the art and, in certain embodiments, can be independently verified by more than one technique, such as, but not limited to, electron microscopy, flow cytometry analysis of extracellular vesicles harboring exogenous fluorescent molecules, nanoparticle tracking analysis, resistive pulse sensing, and determination of total protein concentrations.

### Further Assessments and Characterizations of Extracellular Vesicle Preparations

The identity and concentration of the extracellular vesicles in a preparation or fraction and/or the quantification standard sample can be assessed and/or validated by *in vitro* assays. For example, the identity and concentration of the extracellular vesicles is determined by counting the number of complexes in a population, e.g., by microscopy, by flow cytometry, or by hemacytometry. Alternatively, or in addition, the identity and/or concentration of the extracellular vesicles is assessed by analysis of protein content of the complex, e.g., by flow cytometry, Western blot, immunoprecipitation, fluorescence spectroscopy, chemiluminescence, mass spectrometry, or absorbance spectroscopy. In an embodiment, the protein content assayed is a non-surface protein, e.g., an integral membrane protein, hemoglobin, adult hemoglobin, fetal hemoglobin, embryonic hemoglobin, or a cytoskeletal protein. In an embodiment, the protein content assayed is a surface protein, *e.g.,* a differentiation marker, a receptor, a co-receptor, a transporter, a glycoprotein. In an embodiment, the surface protein is selected from the list including, but not limited to, glycophorin A, CKIT, transferrin receptor, Band3, Kell, CD45, CD46, CD47, CD55, CD59, CR1. In an embodiment, the identity of extracellular vesicles is assessed by analysis of the receiver content of the vesicle, e.g., by flow cytometry, Western blot, immunoprecipitation, fluorescence spectroscopy, chemiluminescence, mass spectrometry, or absorbance spectroscopy. For example, the identity of the extracellular vesicles can be assessed by the mRNA content of the complexes, e.g., by RT-PCR, flow cytometry, or northern blot. The identity of the extracellular vesicles can be assessed by nuclear material content, e.g., by flow cytometry, microscopy, or southern blot, using, *e.g.,* a nuclear stain or a nucleic acid probe. Alternatively, or in addition, the identity of the extracellular vesicles is assessed by lipid content of the complexes, e.g., by flow cytometry, liquid chromatography, or by mass spectrometry.

In some embodiments, the identity of the extracellular vesicles is assessed by metabolic activity of the complexes, e.g., by mass spectrometry, chemiluminescence, fluorescence spectroscopy, absorbance spectroscopy. Metabolic activity can be assessed by ATP consumption rate and/or the metabolic activity is assessed measuring 2,3-diphosphoglycerate (2,3-DPG) level in the parent cells or extracellular vesicles. The metabolic activity can be assessed as the rate of metabolism of one of the following, including but not limited to, acetylsalicylic acid, n-acetylcystein, 4-aminophenol, azathioprine, bunolol, captopril, chlorpromazine, dapsone, daunorubicin, dehydroepiandrosterone, didanosin, dopamine, epinephrine, esmolol, estradiol, estrone, etoposide, haloperidol, heroin, insulin, isoproterenol, isosorbide dinitrate, LY-217896, 6-mercaptopurine, misonidazole, nitroglycerin, norepinephrine, para-aminobenzoic acid. In some embodiments, the identity of the extracellular vesicles is assessed by partitioning of a substrate by the complexes, e.g., by mass spectrometry, chemiluminescence, fluorescence spectroscopy, or absorbance spectroscopy. The substrate can be one of the following, including but not limited to, acetazolamide, arbutine, bumetamide, creatinine, darstine, desethyldorzolamide, digoxigenin digitoxoside, digoxin-16'-glucuronide, epinephrine, gentamycin, hippuric acid, metformin, norepinephrine, p-aminohippuric acid, papaverine, penicillin g, phenol red, serotonin, sulfosalicylic acid, tacrolimus, tetracycline, tucaresol, and vancomycin.

In some embodiments, the extracellular vesicles are assessed for their basic physical properties, e.g., size, mass, volume, diameter, buoyancy, density, and membrane properties, e.g., viscosity, deformability fluctuation, and fluidity. In an embodiment, the diameter of the extracellular vesicles is measured by microscopy or by automated instrumentation, e.g., a hematological analysis instrument or by resistive pulse sensing. In some embodiments, the extracellular vesicle has a longest dimension ranges from about 20-300 nm, such as from about 20-290 nm, 20-280 nm, 20-270 nm, 20-260 nm, 20-250 nm, 20-240 nm, 20-230 nm, 20-220 nm, 20-210 nm, 20-200 nm, 20-190 nm, 20-180 nm, 20-170 nm, 20-160 nm, 20-150 nm, 20-140 nm, 20-130 nm, 20-120 nm, 20-110 nm, 20-100 nm, 20-90 nm, 20-80 nm, 20-70 nm, 20-60 nm, 20-50 nm, 20-40 nm, 20-30 nm, 30-300 nm, 30-290 nm, 30-280 nm, 30-270 nm, 30-260 nm, 30-250 nm, 30-240 nm, 30-230 nm, 30-220 nm, 30-210 nm, 30-200 nm, 30-190 nm, 30-180 nm, 30-170 nm, 30-160 nm, 30-150 nm, 30-140 nm, 30-130 nm, 30-120 nm, 30-110 nm, 30-100 nm, 30-90 nm, 30-80 nm, 30-70 nm, 30-60 nm, 30-50 nm, 30-40 nm, 40-300 nm, 40-290 nm, 40-280 nm, 40-270 nm, 40-260 nm, 40-250 nm, 40-240 nm, 40-230 nm, 40-220 nm, 40-210 nm, 40-200 nm, 40-190 nm, 40-180 nm, 40-170 nm, 40-160 nm, 40-150 nm, 40-140 nm, 40-130 nm, 40-120 nm, 40-110 nm, 40-100 nm, 40-90 nm, 40-80 nm, 40-70 nm, 40-60 nm, 40-50 nm, 50-300 nm, 50-290 nm, 50-280 nm, 50-270 nm, 50-260 nm, 50-250 nm, 50-240 nm, 50-230 nm, 50-220 nm, 50-210 nm, 50-200 nm, 50-190 nm, 50-180 nm, 50-170 nm, 50-160 nm, 50-150 nm, 50-140 nm, 50-130 nm, 50-120 nm, 50-110 nm, 50-100 nm, 50-90 nm, 50-80 nm, 50-70 nm, 50-60 nm, 60-300 nm, 60-290 nm, 60-280 nm, 60-270 nm, 60-260 nm, 60-250 nm, 60-240 nm, 60-230 nm, 60-220 nm, 60-210 nm, 60-200 nm, 60-190 nm, 60-180 nm, 60-170 nm, 60-160 nm, 60-150 nm, 60-140 nm, 60-130 nm, 60-120 nm, 60-110 nm, 60-100 nm, 60-90 nm, 60-80 nm, 60-70 nm, 70-300 nm, 70-290 nm, 70-280 nm, 70-270 nm, 70-260 nm, 70-250 nm, 70-240 nm, 70-230 nm, 70-220 nm, 70-210 nm, 70-200 nm, 70-190 nm, 70-180 nm, 70-170 nm, 70-160 nm, 70-150 nm, 70-140 nm, 70-130 nm, 70-120 nm, 70-110 nm, 70-100 nm, 70-90 nm, 70-80 nm, 80-300 nm, 80-290 nm, 80-280 nm, 80-270 nm, 80-260 nm, 80-250 nm, 80-240 nm, 80-230 nm, 80-220 nm, 80-210 nm, 80-200 nm, 80-190 nm, 80-180 nm, 80-170 nm, 80-160 nm, 80-150 nm, 80-140 nm, 80-130 nm, 80-120 nm, 80-110 nm, 80-100 nm, 80-90 nm, 90-300 nm, 90-290 nm, 90-280 nm, 90-270 nm, 90-260 nm, 90-250 nm, 90-240 nm, 90-230 nm, 90-220 nm, 90-210 nm, 90-200 nm, 90-190 nm, 90-180 nm, 90-170 nm, 90-160 nm, 90-150 nm, 90-140 nm, 90-130 nm, 90-120 nm, 90-110 nm, 90-100 nm, 100-300 nm, 110-290 nm, 120-280 nm, 130-270 nm, 140-260 nm, 150-250 nm, 160-240 nm, 170-230 nm, 180-220 nm, or 190-210 nm.

In particularly preferred embodiments, the extracellular vesicle described herein has a longest dimension ranging from about 30-100 nm. In another preferred embodiment, the extracellular vesicle has a longest dimension ranging from about 20-300 nm. In another preferred embodiment, the extracellular vesicle has a longest dimension ranging from about 40-200 nm. In another embodiment, a population of the extracellular vesicles described herein comprise a population wherein 90% of the extracellular vesicles have a longest dimension of 20-300 nm. In another embodiment, a population of the extracellular vesicles described herein comprise a population wherein 95% of the extracellular vesicles have a longest dimension of 20-300 nm. In another embodiment, a population of the extracellular vesicles described herein comprise a population wherein 99% of the extracellular vesicles have a longest dimension of 20-300 nm. In another embodiment, a population of the extracellular vesicles described herein comprise a population wherein 90% of the extracellular vesicles have a longest dimension of 40-200 nm. In another embodiment, a population of the extracellular vesicles described herein comprise a population wherein 95% of the extracellular vesicles have a longest dimension of 40-200 nm. In another embodiment, a population of the extracellular vesicles described herein comprise a population wherein 99% of the extracellular vesicles have a longest dimension of 40-200 nm. In other preferred embodiments, the size of the extracellular vesicles or population of extracellular vesicles described herein is measured according to methods described, *infra.*

In an embodiment, the average buoyant mass of the extracellular vesicles (pg/cell) is measured using a suspended microchannel resonator or a double suspended microchannel resonator *(see e.g.,* Byun et al PNAS 2013 110(19):7580 and Bryan et al. Lab Chip 2014 14(3):569). In an embodiment, the dry density of the extracellular vesicles is measured by buoyant mass in an H2O-D2O exchange assay *(see e.g.,* Feijo Delgado et al., PLOS One 2013 8(7):e67590). In some embodiments, the extracellular vesicles have an average membrane deformability fluctuation of standard deviation greater than 10, 20, 30, 40, 50, 60, 70, 80, 90, 100 mrad or greater than 100 mrad as measured by spatial light interference microscopy (SLIM) *(see e.g.,* Bhaduri et al., Sci Reports 2014, 4:6211). In an embodiment, the average membrane viscosity of a population of extracellular vesicles is measured by detecting the average fluorescence upon incubation with viscosity-dependent quantum yield fluorophores *(see e.g.,* Haidekker et al. Chem & Biol 2001 8(2):123). In an embodiment, the membrane fluidity of the extracellular vesicles is measured by fluorescence polarization, e.g., with BMG Labtech POLARstar^{®} Omega microplate reader.

### Loading ofExtracellular Vesicles

In certain embodiments, extracellular vesicles produced by the methods described herein comprise a payload. In certain embodiments, the payload is a therapeutic agent. The payload can be loaded into the extracellular vesicles, or adhere to their surface, by any method known in the art. The therapeutic agent can be, but not limited to, a protein (*i.e.,* an antibody or peptide), a small molecule, a nucleic acid, or any other molecule. In certain embodiments, more than one payload is loaded on the extracellular vesicle. In certain embodiments, the extracellular vesicles produced by the methods described herein comprise a receiver *(e.g.,* a targeting moiety). In certain embodiments, the extracellular vesicles comprise both at least one therapeutic agent and a receiver.

### EXAMPLES

Below are examples of specific embodiments for carrying out the present invention. The examples are offered for illustrative purposes only, and are not intended to limit the scope of the present invention in any way. Efforts have been made to ensure accuracy with respect to numbers used *(e.g.,* amounts, temperatures, *etc*.), but some experimental error and deviation should, of course, be allowed for.

The practice of the present invention will employ, unless otherwise indicated, conventional methods of protein chemistry, biochemistry, recombinant DNA techniques and pharmacology, within the skill of the art. Such techniques are explained fully in the literature. See, *e.g.,* T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

### Methods

Fed-Batch (FB) Culturing: A HEK293 SF cell culture was seeded in CDM4PERMAb medium supplemented with 8 mM Gln at a cell density of 0.5E6 viable cells/mL (FB#1 Example 1-3 below) and 0.3E6 viable cells/mL (FB#2 Example 1-3 below), and -100% viability in duplicate shake flasks on culture day 0 using a maintenance cell culture. The shake flasks were agitated at 125 rpm and a shaking angle of 90 degrees from inoculation and until culture harvest on day 8 (FB#1) and day 10 (FB#2), respectively. The cultures were maintained in a shaking incubator set to 37°C, 8% CO₂, and 80% relative humidity. Advanced^{™} CHO Feed 1 was added to the cultures on days 2, 4, 7 (FB#1) or days 3, 5, 7, 9 (FB#2) at 5% of the current culture volume (CVV). In addition, the FB#2 cultures were fed 2.6 g/L of glucose on each feed day. Samples were taken daily for cell density and cell viability from both FB#1 and #2, while exosome titer was estimated only for the FB#2 samples.

Batch-Refeed (BR) Culturing: Batch-refeed method was used to simulate perfusion at a high-throughput scale and to maintain high cell viability and culture health over extended period. The batch-refeed cell culture model has been previously shown to approximate perfusion closely [A. Villiger-Oberbek et al., 2015, J Biotechnol 212(10); 21-29]. A batch-refeed protocol capable of supporting high cell densities was used. A HEK293 SF cell culture was seeded in CDM4PERMAb medium supplemented with 8 mM Gln at a cell density of approximately 0.5E6 viable cells/mL (BR#1 Example 1-3, BR#3a and BR#3b in Example 4-6 below) and 0.3E6 viable cells/mL (BR#2 Example 1-3 below), and -100% viability in duplicate a) shake flasks (BR#1, 2), or b) spin tubes (BR#3a, 3b) on culture day 0 from a maintenance cell culture. The shake flasks and spin tubes were agitated at 125 and 220 rpm, respectively, and at a shaking angle of 90 degrees from inoculation and until culture harvest on day 16 (BR#1), day 13 (BR#2), and day 21 (BR#3a and 3b), correspondingly. All cultures were maintained in a shaking incubator set to 37°C, 8% CO₂, and 80% relative humidity. In all culturing vessels, 100% of the culture medium was exchanged with fresh medium daily at approximately 22-24 h intervals starting from culture day 3 and until the end of each experiment. To that end, the shake flasks cultures were transferred to centrifuge tubes and spun down to remove the spent media, which is also when titer samples were collected for BR#2 cultures. All pellets were then resuspended in the appropriate amount of fresh medium upon transfer to the shake flasks for further culturing. The spin tube flasks were spun down directly in the culturing vessel and the pellets were resuspended *in situ.* Titer samples were collected from the spent medium of both BR#3a and BR#3b. For all cultures, cell density and viability measurements were taken once daily, prior to media exchange. In addition, the BR#3a and BR#3b cultures were bled starting from culture day 8 and 10, respectively, to maintain the cultures at the corresponding target viable cell density of 30 and 40E6 cells/mL.

Perfusion Bioreactor (PB) Cell Culture with Alternating Tangential Flow Filtration: The perfusion process was carried out in 5L Applikon glass bioreactors using a Finesse G3 Lab Universal Controllers. The reactors were configured with a cell retention device with ATF technology (Repligen). Prior to autoclaving, the reactor DO and pH probes were prepared by a two-point calibration. On the day prior to inoculation, the reactor was batched with CDM4PERMAb medium supplemented with 8 mM Gln and 2 g/L pluronic, which was then held overnight at 37°C. One-point DO and pH calibrations were carried out prior to inoculation the following day. The filter was then primed with media and the vessel was inoculated with exponentially growing HEK293 SF maintenance culture at -100% viability and targeting 0.3E6 viable cells/mL (PB#1 Example 7 below) and 0.6E6 viable cells/mL (PB#2 Example 7-8 below). For both PB#1 and PB#2, perfusion was started at one reactor volume per day (RV/d) on day 3 of the process, mimicking the batch-refeed experimental setup. Of note, perfusion rate in PB#1 culture was increased to 1.5 RV/d on culture day 6 and maintained at this rate until harvest. Perfusion was stopped at reactor harvest. During both runs, samples were taken daily for cell density and cell viability, and from PB#2 culture at predefined intervals for exosome titer estimation, either directly from the reactor, or from the permeate stream over the duration of each culture.

Tangential Flow Filtration Perfusion Cell Culture: The tangential flow filtration (TFF) perfusion process (Example 9) was carried out in two 5L Applikon glass bioreactors using Finesse G3 Lab Universal Controllers. The reactors were configured with the 2-5 µm pore size, 65 cm² Spectrum Sampler hollow fiber filter (PN S06-R02M-03-1L-N, Spectrum Laboratories, Inc., Rancho Dominguez, CA), using the Levitronix PuraLev^{®} i30SU single-use pump system (Levitronix GmbH, Zurich, Switzerland) operating at recirculation rates of 300-400 mL/min for the recirculation loop (Figure 12). The day of inoculation, the reactors were batched with CDM4PERMAb supplemented with 8 mM Gln and 2 g/L pluronic. One-point DO and pH calibrations were carried out prior to inoculation. The filter was then primed with media, and the vessels were inoculated with exponentially growing HEK293 SF maintenance culture at -100% viability, targeting 0.3E6 viable cells/mL. Perfusion was started at one reactor volume per day (RV/d) on day 2 of the process. The perfusion media used was CDM4PERMAb supplemented with 12 mM Gln, 3 g/L glucose, and 2 g/L pluronic. After 7 days of growth, cell bleeds of approximately 30-40% reactor volume were initiated to maintain cell density at a target 40E6 cells/mL. Permeate was pumped through a two-filter clarification train using the Sartoclean^{®} 3 0.8 µm (PN 5605304E7--OO-A, Sartorius Stedim North America Inc, Bohemia, NY) and the Sartopore^{®} 2 0.45 µm (PN 5445306G8--SS-A, Sartorius Stedim North America Inc.) filters in series. Samples were taken from the bioreactor, post-hollow fiber, post-Sartoclean^{®}, and post-Sartopore^{®} 2 to determine process yield across each filter via AEX titer. Both reactors were maintained at steady state until the experiment was terminated on day 28. After day 7, antifoam was added daily to the cultures to knock down foam layers that would accumulate.

EV Harvesting: For EV titer estimation, the fed-batch samples were spun down at 1600 × g for 10 min to remove cells and the supernatant was passed through a 0.8 µm filter to assure complete removal of cells and larger cell debris. The batch-refeed samples were taken once the cultures were spun down at 800-1500 × g for 5 min to recover spent medium prior to fresh medium addition. The samples were further passed through the 0.8 µm filter. For titer measurements in the perfusion bioreactor cultures, the samples were pulled directly from the reactor and from the permeate stream over the duration of each culture. Samples from the reactor were then spun down at 1600 × g for 10 min to remove cells. The supernatant then undergone an additional 0.8 µm filtration step. The permeate samples were passed through the 0.8 µm filter without prior centrifugation.

To estimate the titer of exosomes from clarified cell culture harvest, samples were analyzed by one-dimensional liquid chromatography, by examining the intrinsic fluorescence properties of the exosomes. Methods for estimating the titer of exosomes using intrinsic fluorescence is described in detail in International Patent Application PCT/US2017/066324, which is incorporated herein by reference in its entirety. Briefly, exosome samples were applied to an anion exchange chromatography (AEX) column, and eluted in a linear gradient from 0 M to 2 M NaCl. Eluted fractions were analyzed by fluorescence excitation at 556 nm, and monitoring fluorescence emission at 573 nm. The area under the exosome peak, which elutes as a single fluorescent species at ~8.5 minutes, is linearly correlated with the exosome titer of the sample as determined by NTA.

### Example 1: Culturing in chemically-defined culture medium yields high cell densities, and batch re-feed culturing yields higher cell densities than fed-batch cultures

Cell culture growth obtained in simulated perfusion cultures was compared to that obtained in fed-batch cultures. Cultures were inoculated at ~0.3-0.5E6 viable cells/mL with HEK293 SF cells in shake flasks in the same basal cell culture medium and cultured either in fed-batch (FB) or batch-refeed (BR) mode for up to 16 days. Starting from day three, feed was added to the fed-batch cultures as described in the Methods section, and the batch-refeed cultures were spun down daily and one reactor volume of spent medium was exchanged with one reactor volume of fresh basal medium (1 RV/d) following cell resuspension. Based on the cell growth results, ~35E6 cells/mL were successfully maintained for about a week in batch-refeed at >85% viability, while only about 12-14E6 cells/mL could be reached in fed-batch before cell viabilities dropped below 80% (Figures 1A-B).

### Example 2: Batch refeed culturing yields higher EV titers than fed-batch cultures

To determine whether the higher cell densities achieved in batch refeed translated to higher cumulative EV titer, EV titer was determined by analysis of intrinsic fluorescence (Figure 2). More importantly, however, the maximum final yield obtained in a 9-day fed-batch was ~3E12 particles/100 mL vessel volume, while maintaining the cultures in batch refeed at approximately 2.5X higher cell density resulted in volumetric productivity of ~1.3E10 EV/mL/d. In a 9-day batch-refeed steady-state process maintaining ~35E6 viable cells/mL, this volumetric productivity would translate to about 3-fold higher EV yield with the batch refeed culture harvest from a vessel volume of the same size as compared to that of the fed-batch (Figure 3).

### Example 3: Batch refeed culturing yields more pure EV harvest profiles than fed-batch cultures

HPLC chromatograms were evaluated for consistency throughout the culture durations. Analysis of anion exchange chromatograms revealed EV harvest profiles with reduced contamination for the batch-refeed culture as compared to fed-batch (Figures 4A-F). First, an increase of smaller/less negatively charged species in the elute was noted starting from day 9 in fed-batch, while a sharp intensity peak continued to elute at ~8.5 min throughout the batch-refeed culture (Figures 4A, D). In addition, more of the larger/more negatively charged species were also measured on day 10 in fed batch (Figure 4A). Secondly, the fluorescence profiles tracking proteinaceous content, including small molecules, metabolites, membranous contaminants, and combinations thereof, in the harvest were almost 10-fold higher in the fed batch compared to batch refeed (Figures 4B, E). Finally, a more diverse population of mostly nucleic acid species and other molecules besides proteins, such as small molecules, metabolites, membranous contaminants and combination thereof, of both smaller and higher negative charge was detected in the fed-batch harvest, starting as early as day 6 (Figure 4C). In contrast, a largely single peak eluted in batch-refeed harvest up until day 9, when a small population of more negatively charged species started to appear (Figure 4F). This can potentially be further minimized or even avoided with the optimization of batch-refeed, and perfusion, parameters. Taken together, the data suggests less contamination of the batch refeed harvest with both lysed EVs and aggregates thereof, as well as proteinaceous material and nucleic acids, small molecules, metabolites, membranous contaminants, and combinations thereof released from dying and lysed cells, as the changes in the chromatogram profiles were concomitant with changes in cell viability.

### Example 4: Batch refeed with cell bleeds to maintain target density improves culture viability

Cell culture growth obtained in simulated perfusion cultures maintained at either 30E6 or 40E6 cells/mL was compared. Cultures were inoculated at 0.5E6 viable cells/mL with HEK293 SF cells in spin tubes in the same basal cell culture medium and cultured in batch-refeed mode starting from day 3 and up to 21 days, as described above in the Methods section. When the cultures reached their respective target densities on day 8 and day 10, respectively, manual cell bleeds were applied to maintain the VCD at a semi steady state until the end of the run. Based on the cell growth results, cell viability of the cultures maintained at ~30E6 cells/mL for 14 days remained above 95%, while cell viability in the cultures kept at ~40E6 cells/mL was 90% and greater (Figures 5A-B).

### Example 5: Target viable cell denistv maintained at steady state impacts exosome yield

To determine whether the higher cell densities maintained at steady state in batch-refeed cultures with bleeds translated to higher EV yield, EV titer was determined by analysis of intrinsic fluorescence (Figure 6). The average VPR measured in the cultures maintained at 30E6 cells/mL was about 2E10 EV/mL/d and about 3E10 EV/mL/d for the cultures maintained at 40E6 cells/mL (Figure 7). This translated to the final yield obtained from each 12-day steady-state process of ~2E12 and 3.5E12 particles/10 mL vessel volume, which resulted in about 0.6-fold increase in exosome yield (Figure 7).

### Example 6: Batch refeed with cell bleeds to maintain target density further decreases harvest contamination with impurities without significant impact on exosome yield

HPLC chromatograms were evaluated for consistency throughout the culture durations. Analysis of anion exchange chromatograms revealed EV harvest profiles with slightly reduced level of contamination for the batch-refeed cultures maintained at 30E6 cells/mL as compared to cultures maintained at 40E6 cells/mL (Figures 8A-F). First, no significant difference in exosome peak profiles eluting at ~8.5 min was noted between the cultures, except for peak intensity, which corresponded to the respective culture densities at steady state (Figures 8A, D). However, about 2-fold increase in proteinaceous content, including small molecules, metabolites, membranous contaminants, and combinations thereof, was measured in the harvest from the batch-refeed cultures maintained at 40E6 cells/mL as compared to those kept at 30E6 cells/mL (Figures 8B, E). Finally, the peak eluting at ~7 min on the chromatograms tracking mostly nucleic acid species and other molecules besides proteins, such as small molecules, metabolites, membranous contaminants and combination thereof, increased in intensity, in addition to a visible shoulder gaining visibility, in the cultures maintained at 40E6 cells/mL as opposed to those at 30E6 cells/mL (Figures 8C, F). These results suggest that by optimizing batch-refeed, and therefore perfusion culture conditions, the composition of culture harvest and its purity can be modulated by adjusting cell culture parameters. Taken together, the data suggests less contamination of the batch refeed harvest collected from cultures maintained at 30E6 cells/mL with both proteinaceous material and nucleic acids, small molecules, metabolites, membranous contaminants, and combinations thereof released from dying and lysed cells, as the changes in the chromatogram profiles were again concomitant with changes in cell viability.

### Example 7: Perfusion bioreactor culture growth corroborates batch refeed results

Cell culture growth obtained in perfusion bioreactor cultures closely followed that of batch-refeed results. Cultures were inoculated at 0.3-0.6E6 viable cells/mL with HEK293 SF cells in glass bioreactors in the same basal cell culture medium and cultured in perfusion mode using alternating tangential flow (ATF) filtration starting from when the cultures reached ~2.5E6 cells/mL (approximately culture day 3), and up to harvest as described in the Methods section. Based on the cell growth results, the perfusion cultures reached about 40E6 cells/mL within a similar time frame as the batch-refeed cultures (Figures 5A and 9A) and maintained even higher cell viabilities, >98% as compared to 90-95% in batch refeed (Figures 5B and 9B).

### Example 8: Cell retention device with filtering technology allows for almost complete passage of exosomes from the perfusion bioreactor and into the harvest stream

Titer samples were collected during the last 4 days of the PB#2 perfusion culture, both directly from the reactor (R) and from the filter-clarified cell culture harvest (H). Analysis of anion exchange chromatograms of the EV intrinsic fluorescence revealed no major difference in exosome peak profile intensities eluting at ~8.5 min from samples collected pre- (R) and post-filter (H) (Figure 10). Considering assay variability, this signified an almost complete passage of exosomes from the bioreactor through the filter and into the harvest, with no apparent exosome retention inside the culturing vessel (Figure 10). Measured EV titer was used to calculate volumetric productivity (VPR), which at 40E6 cells/mL approximated ∼1.1E11 EV/mL/d (Figure 11). This VPR would translate to a 12-day steady-state process yield at that density of about 3E15 particles/2.4 L vessel volume (Figure 11). Compared to batch refeed, 3-10X increase in VPR was noted in perfusion (Figures 3, 7, and 11), underlying its benefits for harvesting labile products, such as exosomes, susceptible to waste accumulation in the culturing vessel, as well as to the different types of lytic enzymes released into the culture medium from dying cells, in addition to other unfavorable cell culture parameters.

### Example 9: Tangential flow filtration perfusion cell culture promotes high density cell culture and significant improvements in exosome production

Tangential flow filtration perfusion cell culture process using wild-type HEK 293 SF cells was carried out in four independent bioreactors at different filtration rates as described in the Methods section for 28 days. Additionally, another tangential flow filtration perfusion cell culture process using HEK293 cells overexpressing full-length PTGFRN-GFP ("Engineered") was carried out as described in the Methods section for 28 days. Cell viability was >95% for the wild-type HEK293 SF cells, and other than a temporary drop in viability for the Engineered HEK293 SF due to increased metabolic demands that were adjusted by increasing agitation rate to improve oxygenation, viability was also maintained at -90% for the Engineered cells. (Figure 13A [average of all wild-type runs] and Figure 13B [individual runs shown], right axis, solid lines). Viable cell density (VCD) was held at ~40E6 cells/ml during the course of the run for both wild-type and Engineered cells (Figure 13A [average of all wild-type runs] and Figure 13B [individual runs shown], left axis, dotted lines). Lactate dehydrogenase (LDH) was retained at a low level (Figures 14A-B) and glucose and lactate were held constant during the run (Figures 15A-B, solid lines and dotted lines, respectively). Glutamine and ammonia levels were also held constant once peak VCD was reached (Figures 16A-B, solid and dotted lines, respectively). pH remained at ~7 throughout the course of the experiment (Figures 17A-B) and pCO₂ was held constant (Figures 18A-B), indicating successful runs in optimal conditions.

Exosome titer was measured during the perfusion bioreactor experiment by AEX HPLC at excitation 556 nm, emission 573 nm as described above in the Methods section. Compared to a fed batch 50 L single use bioreactor (SUB) run for 9 days (50L SUB Fed-Batch), the perfusion bioreactor runs produced significantly more exosomes both in total (Figure 19) and in terms of specific productivity for both natural and Engineered exosomes (Figure 20). The sieving coefficient across the TFF hollow fiber membrane remained near 100%, indicating that exosomes were not being retained in the bioreactor and were successfully passing into the perfusate (Figures 21A-B). Volumetric productivity of the 3.5 L perfusion reactor was substantially greater compared to a fed batch 50 L SUB (Figure 22) and total particle yield was dramatically improved over time as well (Figures 23A-B).

Exosome identity and purity from the perfusion bioreactors were confirmed by electron microscopy (Figure 24). Exosomes from fed-batch and perfusion cultures purified by Optiprep^{™} density-gradient ultracentrifugation (as described elsewhere, e.g., International Patent Application No. PCT/US2017/066324) appeared qualitatively similar, confirming the presence of exosomes in the perfusate. To further demonstrate the presence of exosomes in the perfusate, exosomes from PTGFRN-overexpressing cells grown in fed-batch and perfusion bioreactors were subjected to proteomic analysis (as described in International Patent Application No. PCT/US2018/046997). The identity and relative abundance of exosome proteins were comparable between the two preparations (Figure 25), demonstrating the presence of *bona fide* exosomes prepared by perfusion culture methods.

While the invention has been particularly shown and described with reference to a preferred embodiment and various alternate embodiments, it will be understood by persons skilled in the relevant art that various changes in form and details can be made therein without departing from the spirit and scope of the invention.

All references, issued patents and patent applications cited within the body of the instant specification are hereby incorporated by reference in their entirety, for all purposes.

In addition, the invention further comprises the following numbered embodiments.
**1.** A method of producing extracellular vesicles, comprising:
   culturing a population of producer cells in single-cell suspension, wherein the cells are cultured in chemically-defined culture medium, wherein the culture medium lacks animal-derived serum and animal-derived components; and
   obtaining from the cell culture an extracellular vesicle preparation comprising extracellular vesicles.
**2.** The method of embodiment 1, wherein viability of the cells after 8 days in culture is greater than 98%.
**3.** The method of embodiment 1, wherein viability of the cells after 21 days in culture is greater than 90%.
**4.** The method of embodiment 3, wherein the viability of the cells after 21 days in culture is greater than 95%.
**5.** The method of any one of the above embodiments, wherein the volumetric productivity of extracellular vesicles is greater than 1 × 10¹⁰ extracellular vesicle particles/ml culture /day.
**6.** The method of embodiment 5, wherein the volumetric productivity of extracellular vesicles is greater than 1 × 10¹¹ extracellular vesicle particles/ml culture /day.
**7.** The method of any one of the above embodiments, wherein the yield of extracellular vesicles is greater than 3 × 10¹⁵ extracellular vesicle particles/2.4L reactor volume/12-day culture duration.
**8.** The method of any one of the above embodiments, wherein the culturing is performed using a fed-batch culturing method.
**9.** The method of any one of embodiments 1 to 7, wherein the culturing is performed using a batch refeed culturing method.
**10.** The method of any one of embodiments 1 to 7, wherein the culturing is performed using a perfusion culturing method.
**11.** The method of embodiment 10, wherein the perfusion culturing method is tangential flow filtration perfusion.
**12.** The method of embodiment 10, wherein the perfusion culturing method is alternating tangential flow filtration perfusion.
**13.** The method of any one of embodiments 9 to 12, wherein the method results in increased cell viability compared to cells cultured using a fed-batch culturing method cultured for the same number of days.
**14.** The method of embodiment 13, wherein the increased cell viability is two-fold or greater cell viability after 10 days of culture.
**15.** The method of any one of the above embodiments, wherein the extracellular vesicles are fractionated by column chromatography to determine the amount of contaminants in the isolated extracellular vesicles.
**16.** The method of any one of embodiments 9 to 15, wherein the extracellular vesicle preparation comprises reduced proteinaceous contaminants, nucleic acid contaminants, small molecules, metabolites, membranous contaminants, or combinations thereof, compared to extracellular vesicle preparations harvested using a fed-batch culturing method cultured for the same number of days.
**17.** The method of embodiment 16, wherein the reduced proteinaceous contaminants, nucleic acid contaminants, small molecules, metabolites, membranous contaminants, or combinations thereof are less than 60% for first 10 days of culture.
**18.** The method of embodiment 16 or 17, wherein the proteinaceous contaminants are measured by ratio of 280 nm / 350 nm signal.
**19.** The method of embodiments 18, wherein the nucleic acid contaminants are measured by 254 nm signal.
**20.** The method of any one of embodiments 9 to 19, wherein the extracellular vesicle preparation comprises increased abundance of extracellular vesicles, compared to extracellular vesicle preparations harvested using a fed-batch culturing method cultured for the same number of days.
**21.** The method of embodiment 20, wherein the increased abundance of extracellular vesicles is greater than 4-fold after 9 days of culture.
**22.** The method of any one of the above embodiments, wherein the cells are cultured in a culturing vessel.
**23.** The method of embodiment 22, wherein the culturing vessel is a shake flask, a conical tube, a shallow-well multititer plate, a deep-well multititer plate, or a bioreactor.
**24.** The method of embodiment 23, wherein the culturing vessel is a bioreactor.
**25.** The method of embodiment 24, wherein the bioreactor is a microbioreactor, a glass bioreactor, a stainless steel bioreactor, or a single use bioreactor.
**26.** The method of embodiment 24 or 25, wherein the bioreactor is a perfusion bioreactor.
**27.** The method of embodiment 26, wherein the bioreactor is connected to a cell retention device.
**28.** The method of embodiment 27, wherein the bioreactor is connected to a cell retention device with filtering technology.
**29.** The method of any one of the above embodiments, wherein the cells are subjected to mixing during culture.
**30.** The method of embodiment 29, wherein the mixing is shaking, agitation, or stirring.
**31.** The method of embodiment 30, wherein the shaking is performed at a speed of 10 rpm or greater.
**32.** The method of embodiment 31, wherein the shaking is performed at a speed of 125 rpm.
**33.** The method of embodiment 31, wherein the shaking is performed at a speed of 160 rpm.
**34.** The method of embodiment 31, wherein the shaking is performed at a speed of 220 rpm.
**35.** The method of any one of embodiments 30 to 34, wherein the cells are shaken at an angle of 90 degrees.
**36.** The method of any one of embodiments 30 to 34, wherein the cells are shaken at an angle less than 90 degrees.
**37.** The method of embodiment 36, wherein the shaking speed is 160 rpm and cells are shaken at an angle less than 90 degrees.
**38.** The method of any one of the above cells, wherein the extracellular vesicle preparation is fractionated by a chromatography step, wherein the fractions are detected by intrinsic fluorescence of the extracellular vesicles.
**39.** The method of embodiment 38, wherein the intrinsic fluorescence is detected using an excitation wavelength ranging from 450 nm to 650 nm.
**40.** The method of embodiment 39, wherein the intrinsic fluorescence is detected using an excitation wavelength of 556 nm.
**41.** The method of any one of embodiments 38 to 40, wherein the intrinsic fluorescence is detected using an emission wavelength ranging from 500 nm to 650 nm.
**42.** The method of embodiment 41, wherein the intrinsic fluorescence is detected using an emission wavelength of 573 nm.
**43.** The method of any one of embodiments 38 to 42, wherein the chromatography is size-exclusion chromatography.
**44.** The method of any one of embodiments 38 to 42, wherein the chromatography is ion-exchange chromatography.
**45.** The method of embodiment 44, wherein the ion-exchange chromatography is strong anion-exchange chromatography.
**46.** The method of any one of embodiments 38 to 45, wherein the peak fraction from the chromatography step is isolated.
**47.** The method of any one of the above embodiments, wherein the cells are mammalian cells.
**48.** The method of embodiment 47, wherein the cells are Chinese hamster ovary (CHO) cells.
**49.** The method of any one of embodiments 1 to 47, wherein the cells are human cells.
**50.** The method of embodiment 49, wherein the human cells are human kidney cells.
**51.** The method of embodiment 50, wherein the cells are HEK293 cells.
**52.** The method of embodiment 50, wherein the cells are HEK293 SF cells.
**53.** The method of any one of embodiments 1 to 46, wherein the cells are insect cells.
**54.** The method of any one of embodiments 1 to 46, wherein the cells are yeast cells.
**55.** The method of any one of embodiments 1 to 46, wherein the cells are bacteria cells.
**56.** The method of any one of the above embodiments, wherein the cells overexpress an exosome specific protein, thereby generating engineered extracellular vesicles overexpressing the exosome specific protein.
**57.** The method of embodiment 56, wherein the exosome-specific protein is PTGFRN, BSG, IGSF2, IGSF3, IGSF8, ITGB1, ITGA4, SLC3A2, or ATP transporter, or a fragment or variant thereof.
**58.** The method of embodiment 57, wherein the exosome-specific protein is PTGFRN or a fragment or variant thereof.
**59.** The method of embodiment 56, wherein the exosome-specific protein is MARCKS, MARCKSL1, or BASP1, or a fragment or variant thereof.
**60.** The method of any one of the above embodiments, wherein the extracellular vesicles comprise at least one therapeutic agent.
**61.** The method of any one of the above embodiments, wherein the extracellular vesicles are exosomes.

## Claims

1. A method of producing extracellular vesicles, comprising:
culturing a population of producer cells in suspension culture, wherein the cells are cultured in chemically-defined culture medium, wherein the culture medium lacks animal-derived serum and animal-derived components; and
obtaining from the cell culture an extracellular vesicle preparation comprising extracellular vesicles,
wherein the culturing comprises a perfusion culturing method.

2. The method of claim 1, wherein:
(i) the viability of the cells after 8 days in culture is greater than 98% or the viability of the cells after 21 days in culture is greater than 90% or greater than 95%;
(ii) the volumetric productivity of extracellular vesicles is greater than 1 × 10¹⁰ extracellular vesicle particles/ml culture /day or greater than 1 × 10¹¹ extracellular vesicle particles/ml culture /day; and/or
(iii) the yield of extracellular vesicles is greater than 3 × 10¹⁵ extracellular vesicle particles/2.4L reactor volume/12-day culture duration.

3. The method of claim 1 or 2, wherein the perfusion culturing method is a continuous perfusion culturing method, or wherein the perfusion culturing method is tangential flow filtration perfusion or alternating tangential flow filtration perfusion.

4. The method of any one of claims 1 to 3, wherein the method results in increased cell viability compared to cells cultured using a fed-batch culturing method cultured for the same number of days, optionally wherein the increased cell viability is two-fold or greater cell viability after 10 days of culture.

5. The method of any one of the above claims, wherein the extracellular vesicles are fractionated by column chromatography to determine the amount of contaminants in the isolated extracellular vesicles.

6. The method of any one of claims 1 to 5, wherein:
(i) the extracellular vesicle preparation comprises reduced proteinaceous contaminants, nucleic acid contaminants, small molecules, metabolites, membranous contaminants, or combinations thereof, compared to extracellular vesicle preparations harvested using a fed-batch culturing method cultured for the same number of days, optionally wherein the reduced proteinaceous contaminants, nucleic acid contaminants, small molecules, metabolites, membranous contaminants, or combinations thereof are less than 60% for first 10 days of culture; and/or
(ii) the extracellular vesicle preparation comprises increased abundance of extracellular vesicles, compared to extracellular vesicle preparations harvested using a fed-batch culturing method cultured for the same number of days, optionally wherein the increased abundance of extracellular vesicles is greater than 4-fold after 9 days of culture.

7. The method of claim 6, wherein the proteinaceous contaminants are measured by ratio of 280 nm / 350 nm signal and/or wherein the nucleic acid contaminants are measured by 254 nm signal.

8. The method of any one of the above claims, wherein the cells are cultured in a culturing vessel, optionally wherein the culturing vessel is:
(i) a shake flask, a conical tube, a shallow-well multititer plate, a deep-well multititer plate, or a bioreactor;
(ii) a bioreactor, optionally a microbioreactor, a glass bioreactor, a stainless steel bioreactor, or a single use bioreactor;
(iii) a perfusion bioreactor;
(iv) a perfusion bioreactor connected to a cell retention device, optionally a cell retention device with filtering technology.

9. The method of any one of the above claims, wherein the cells are subjected to mixing during culture, optionally wherein the mixing is shaking, agitation, or stirring, wherein:
(i) the shaking is performed at a speed of 10 rpm or greater or at a speed of 125 rpm, 160 rpm, or 220 rpm; and/or
(ii) the cells are shaken at an angle of 90 degrees or at an angle of less than 90 degrees; optionally wherein the shaking speed is 160 rpm and cells are shaken at an angle less than 90 degrees.

10. The method of any one of the above claims, wherein the extracellular vesicle preparation is fractionated by a chromatography step, wherein the fractions are detected by intrinsic fluorescence of the extracellular vesicles, optionally wherein:
(i) the intrinsic fluorescence is detected using an excitation wavelength ranging from 450 nm to 650 nm, optionally using an excitation wavelength of 556 nm; and/or
(ii) the intrinsic fluorescence is detected using an emission wavelength ranging from 500 nm to 650 nm, optionally using an emission wavelength of 573 nm.

11. The method of claim 10, wherein the chromatography is size-exclusion chromatography or ion-exchange chromatography, optionally wherein the ion-exchange chromatography is strong anion-exchange chromatography.

12. The method of claim 10 or 11, wherein the peak fraction from the chromatography step is isolated.

13. The method of any one of the above claims, wherein the cells are:
(i) mammalian cells, optionally Chinese hamster ovary (CHO) cells;
(ii) human cells, optionally human kidney cells, HEK293 cells or HEK293 SF cells;
(iii) insect cells;
(iv) yeast cells; or
(v) bacteria cells.

14. The method of any one of the above claims, wherein the cells overexpress an exosome specific protein, thereby generating engineered extracellular vesicles overexpressing the exosome specific protein, optionally wherein the exosome-specific protein is:
(i) PTGFRN, BSG, IGSF2, IGSF3, IGSF8, ITGB1, ITGA4, SLC3A2, or ATP transporter, or a fragment or variant thereof;
(ii) PTGFRN or a fragment or variant thereof; or
(iii) MARCKS, MARCKSL1, or BASP1, or a fragment or variant thereof.

15. The method of any one of the above claims, wherein the extracellular vesicles comprise at least one therapeutic agent and/or wherein the extracellullar vesicles are exosomes.
